(19) Europäisches Patentamt
European Patent Office
Office européen des brevets

(11) EP 2 474 544 B1

(12) EUROPEAN PATENT SPECIFICATION

(45) Date of publication and mention of the grant of the patent:
**12.07.2017 Bulletin 2017/28**

(21) Application number: **10813754.8**

(22) Date of filing: **02.09.2010**

(51) Int Cl.:
***C07D 417/14*** $^{(2006.01)}$ ***A61K 31/426*** $^{(2006.01)}$
***A61K 31/4439*** $^{(2006.01)}$ ***A61K 31/506*** $^{(2006.01)}$
***A61K 31/5377*** $^{(2006.01)}$ ***A61P 25/22*** $^{(2006.01)}$
***C07D 491/048*** $^{(2006.01)}$ ***C07D 491/052*** $^{(2006.01)}$
***C07D 495/04*** $^{(2006.01)}$

(86) International application number:
**PCT/JP2010/064989**

(87) International publication number:
**WO 2011/027806 (10.03.2011 Gazette 2011/10)**

(54) **THERAPEUTIC AGENT FOR ANXIETY DISORDERS**

THERAPEUTISCHER WIRKSTOFF FÜR ANGSTSTÖRUNGEN

AGENT THÉRAPEUTIQUE POUR DES TROUBLES DE L'ANXIÉTÉ

(84) Designated Contracting States:
**AL AT BE BG CH CY CZ DE DK EE ES FI FR GB GR HR HU IE IS IT LI LT LU LV MC MK MT NL NO PL PT RO SE SI SK SM TR**
Designated Extension States:
**BA ME RS**

(30) Priority: **02.09.2009 JP 2009202894**

(43) Date of publication of application:
**11.07.2012 Bulletin 2012/28**

(73) Proprietor: **Kyowa Hakko Kirin Co., Ltd.**
**Tokyo 100-8185 (JP)**

(72) Inventors:
- **KASE, Junya**
  **Tokyo 100-8185 (JP)**
- **KANDA, Tomoyuki**
  **Shizuoka 411-8731 (JP)**

(74) Representative: **Vossius & Partner**
**Patentanwälte Rechtsanwälte mbB**
**Siebertstrasse 3**
**81675 München (DE)**

(56) References cited:
**EP-A1- 1 700 856**
**EP-A1- 1 894 930**
**WO-A1-00/69464**
**WO-A1-03/022283**
**WO-A1-2004/108137**
**WO-A1-2005/063743**
**WO-A1-2007/015528**

- **GRIEBEL, G. ET AL.: 'Comparison of the behavioral effects of an adenosine A1/A2-receptor antagonist, CGS 15943A, and an Al-selective antagonist, DPCPX' PSYCHOPHARMACOLOGY vol. 103, no. 4, 1991, BERLIN, GERMANY, pages 541 - 4, XP008154902**
- **FLORIO CHIARA ET AL: "Adenosine A1 receptors modulate anxiety in CD1 mice", PSYCHOPHARMACOLOGY, vol. 136, no. 4, April 1998 (1998-04), pages 311-319, ISSN: 0033-3158**
- **JACOBSON KENNETH A ET AL: "Adenosine receptors as therapeutic targets.", NATURE REVIEWS. DRUG DISCOVERY MAR 2006, vol. 5, no. 3, March 2006 (2006-03), pages 247-264, ISSN: 1474-1776**

Note: Within nine months of the publication of the mention of the grant of the European patent in the European Patent Bulletin, any person may give notice to the European Patent Office of opposition to that patent, in accordance with the Implementing Regulations. Notice of opposition shall not be deemed to have been filed until the opposition fee has been paid. (Art. 99(1) European Patent Convention).

EP 2 474 544 B1

Printed by Jouve, 75001 PARIS (FR)

## Description

Technical Field

The present invention relates to an agent for the treatment and/or prophylaxis of an anxiety disorder such as panic disorder, agoraphobia, obsessive-compulsive disorder, social phobia, posttraumatic stress disorder, particular phobia, generalized anxiety disorder or the like.

Background Art

Anxiety disorder

Anxiety disorders are a class of psychological problems, important features of which include excess anxiety, fears, concern, avoidance, forced rituals and the like, and these can cause excess incidence rates, overuse of medical care services, and functional impairment, or result in such outcomes. These are one of the most common psychomedical conditions in the US and almost all other countries. The incidence rate for this disorder is fairly uniform across different cultures. Mostly, females are more likely to experience anxiety disorders than males. Chronic anxiety disorders can raise the cardiovascular mortality rate, so that the appropriate diagnosis and quick treatment initiation are required.

The anxiety disorders listed in the "Diagnostic and Statistical Manual of Mental Disorders" (4th edition - revised in 1994, published by the American Psychiatric Association, Washington D.C., US, p.393-444) include panic disorder with or without agoraphobia, agoraphobia without history of panic disorder, social phobia, obsessive-compulsive disorder (OCD), posttraumatic stress disorder (PTSD), acute stress disorder, generalized anxiety disorder (GAD), an anxiety disorder resulting from general medical conditions, drug-induced anxiety disorder, specific phobias, non-specified anxiety disorders and the like.

Panic disorder with or without agoraphobia

Panic disorders are a type of anxiety disorders, an essential feature of which is recurrent panic attacks (that is, a discrete period of intense fear or discomfort with at least four characteristic related symptoms). Attacks normally last for several minutes (or, in rare cases, several hours) and are unforeseeable. And it does not have the tendency for occurring before exposure to a situation that nearly always causes anxiety, or immediately after exposure, as in the case of simple phobia. This "unforeseeable" aspect of attacks is an essential feature of this type of disorders. A panic attack begins with the sudden occurrence of intense anxiety or fear, and is typically accompanied by physical symptoms, for example, short breaths, vertigo, syncope, choking, palpitation, tremors, sweating, ague, nausea, numbness, hot sensation or chilling, chest pain, and the like. Panic disorders are sometimes associated with agoraphobia; in serious cases, the affected person substantially refuses to leave his or her house.

In clinical samples, agoraphobia is encountered at a higher rate, whereas in local social samples, about one-third to half of persons diagnosed with panic disorders are also suffering agoraphobia.

Agoraphobia without history of panic disorder

Agoraphobia is a condition characterized by anxiety about being in a place or situation which it may be difficult (or embarrassing) to escape, or where it can be impossible to seek help, in the event of a panic attack or a panic-like symptom (for example, fear of the onset of sudden attacks of vertigo or sudden attacks of diarrhea). Agoraphobia occurs in a situation where there are neither panic disorders with agoraphobia nor history of panic disorders. Except that the object of fear resides in the onset of attacks of limited symptoms, rather than immobility-causing or extremely embarrassing panic-like symptoms or complete panic attacks, the essential feature of agoraphobia without history of panic disorders is the same as the feature of panic disorders with agoraphobia.

Obsessive-compulsive disorder (OCD)

Primary symptoms of obsessive-compulsive disorders are completely severe because they can cause distress, take much time, or considerably hamper people’s normal daily activity or lifestyle, and they are recurrent obsessions (that is, thoughts, images or urges that are recurrent, and endure and cause remarkable anxiety) and/or compulsions (that is, repetitive behaviors or mental actions executed to modify anxiety caused by the affected person’s obsessions). Obsessions are typically related to pollution, doubt (including loss of self-confidence), and blasphemy of sexual or religious thought. Typical compulsions include the act of washing, the act of confirmation, the act of arranging objects in order, the act of counting objects and the like.

Social phobia

Social phobia is characterized by an obstinate social or public fear that can be embarrassing. Typical situations which a person with social phobia fears or avoids include parties, meetings, taking a meal in the presence of other person, writing in the presence of other person, speeches, conversations, the first meeting with a non-acquaintance, other related situations and the like. Being exposed to a social or public situation not only causes immediate anxiety reactions, but also almost always causes sweating, tremors, throbbing or striking heartbeats, mental confusion, and the desire to run away.

Posttraumatic stress disorder (PTSD)

Major characteristic symptoms include re-experience of an event due to posttraumatic stress (that is, psychological agony), escape of stimuli that recall the event, general reactive paralysis, increased arousal, and the like. Related "events" include, for example, mere bereavement, chronic disease, marital friction and the like.

Generalized anxiety disorder (GAD)

GADs are essentially characterized by a state where unrealistic or excess anxiety and two or more living environmental concerns last for 6 months or more. There are more days on which the sufferer realizes it difficult to control this situation and is annoyed by the concerns than those without. Signs such as motor tension, autonomic hyperactivity, vigilance, and scanning are clearly manifested.

Particular phobia

Specific phobias are anxiety disorders, the essential feature of which is remarkable fear of limited stimulus (a circumscribed stimulus); this limited stimulus can be said to be an object or situation other than the fear of experiencing a panic attack, getting ashamed, or being embarrassed, in a social situation (this is classified under social phobias). For example, airplane phobia, acrophobia, zoophobia, trypanophobia, hemophobia and the like can be exemplified.

Regarding anxiety disorders, a wide variety of causes are suspected; in particular, genetic temperaments, growth and development in infancy and childhood, as well as combinations thereof with later life experiences are suspected. Anxiety disorders are treated by using counseling, psychotherapy, pharmacological therapy (drug therapy) and the like singly or in combination. Drugs that are typically used to treat anxiety disorder patients include benzodiazepine; selective serotonin reuptake inhibitors (SSRIs), serotonin/noradrenaline uptake inhibitors (SNRIs), buspirone, and the like.

Benzodiazepine belongs to a major class of relatively safe, widely prescribed drugs having quick and adequate anxiolytic effects and sedative hypnotic effects. Drugs belonging to the class of SSRIs and SNRIs are used for the treatment of, for example, anxiety disorders such as panic disorder, agoraphobia, OCD, social phobia, posttraumatic stress disorder, specific phobias, and a broader range of anxiety disorders [Kaplan & Sadock's Comprehensive textbook of psychiatry 7th. edition, 1, p.1441 (1999)]. Buspirone, a relatively selective $5HT_{1A}$ partial agonist, is approved by the FDA as the most useful anxiolytic drug for the treatment of GAD, and is currently frequently used as an auxiliary for SSRIs [Kaplan & Sadock's Comprehensive textbook of psychiatry 7th. edition, 1, p.1441 (1999)].

On the other hand, it is known that adenosine is widely distributed in the whole body, and exhibits a variety of physiological actions on the central nervous system, the cardiac muscle, the kidneys, the smooth muscle, and the like through its receptors (see non-patent document 1).

For example, adenosine $A_1$ antagonists are known to facilitate defecation (Jpn. J. Pharmacol., Vol.68, p.119 (1995)). Further, the adenosine $A_{2A}$ receptors are known to be involved particularly in the central nervous system, and the antagonists of the adenosine $A_{2A}$ receptors are known to be useful as, for example, therapeutic drugs for Parkinson's disease (see non-patent document 2), therapeutic drugs for sleep disturbance (see Nature Neuroscience, p. 858 (2005); patent document 3), therapeutic drugs for depression (Neurology, 61(11 Suppl 6), S82-7 (2003)) and the like. There are many reports concerning the relationship between adenosine receptors and Parkinson's disease (Nature Reviews Drug Discovery, 5, p.845 (2006); Current Pharmaceutical Design, 14, p.1475 (2008)).

Regarding the association between adenosine $A_{2A}$ receptors and depressive symptoms, an investigation using mice deficient in adenosine $A_{2A}$ receptors led to a report that adenosine $A_{2A}$ receptor antagonizing action induces behavioral pharmacological changes similar to those with administration of antidepressants (Br. J. Pharmacol., 134, p.68 (2001)). Xanthine compounds possessing adenosine $A_{2A}$ receptor antagonizing activity are known to possess antidepressive activity (for example, WO94/01114), and are known to further possess anti-Parkinsonian activity (for example, Ann. Neurol., 43, p. 507 (1998)), therapeutic effects on anxiety disorders (for example, WO2004/108137), suppressing activity against neurodegeneration (for example, WO99/12546) and the like. Combinations of adenosine

$A_{2A}$ receptor antagonists and antidepressants or anxiolytic drugs have been reported (see Patent Document 1).

On the other hand, for example, compounds represented by the formulas (IA), (IB), (IC), (ID) and the like are known to have affinity to adenosine $A_{2A}$ receptors and have a therapeutic effect for Parkinson's disease (see patent document 2). It is also known that these compounds are useful as an agent for the treatment and/or prophylaxis of sleep disturbance (see patent document 3).

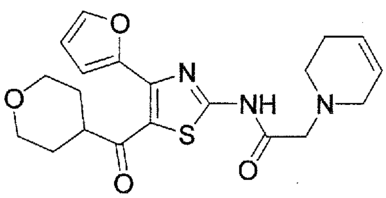

( IA )

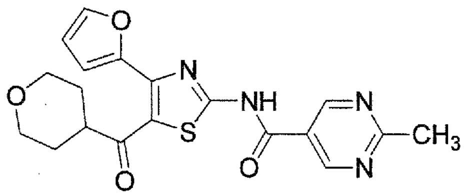

( IB )

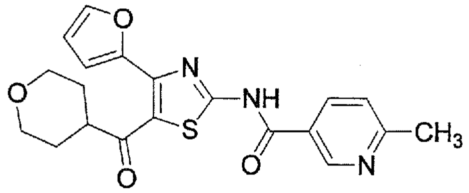

( IC )

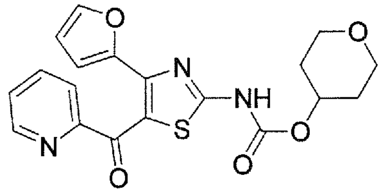

( ID )

Document List

patent documents

EP 1 894 930 discloses thiazole derivatives or pharmaceutically acceptable salts thereof having an adenosine $A_{2A}$ receptor antagonistic activity for use in the treatment or prevention of diseases associated with adenosine $A_{2A}$ receptor.

patent document 1: WO2003/022283
patent document 2: WO2005/063743 (EP 1 700 856)
patent document 3: WO2007/015528

non-patent documents

non-patent document 1: Nature Reviews Drug Discovery, 2006, vol. 5, p. 247
non-patent document 2: Progress in Neurobiology, 2007, vol. 83, p. 332

SUMMARY OF THE INVENTION

Problems to be Solved by the Invention

An object of the present invention is to provide an agent for use in the treatment and/or prophylaxis of an anxiety disorder such as panic disorder, agoraphobia, obsessive-compulsive disorder, social phobia, posttraumatic stress disorder, particular phobia, generalized anxiety disorder or the like.

Means of Solving the Problems

The present application discloses the following (1) - (14).

(1) An agent for use in the treatment and/or prophylaxis of an anxiety disorder, comprising a thiazole derivative

represented by the formula (I)

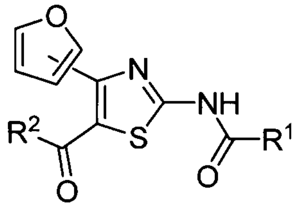

( I )

wherein $R^1$ represents aryl, aralkyl, an aromatic heterocyclic group, aromatic heterocyclyl-alkyl, aliphatic heterocyclyl-alkyl or tetrahydropyranyloxy, each of which is optionally substituted by 1 to 3 substituents selected from the group consisting of halogen; lower alkyl optionally substituted by lower alkoxy or morpholino; lower alkoxy; lower alkanoyl; and vinyl, and $R^2$ represents pyridyl or tetrahydropyranyl, or a pharmaceutically acceptable salt thereof as an active ingredient.

(2) The agent for use in the treatment and/or prophylaxis of an anxiety disorder, comprising the thiazole derivative or the pharmaceutically acceptable salt thereof of (1), wherein $R^1$ is phenyl, pyridyl, pyrimidinyl, 5,6-dihydro-2H-pyridylmethyl or tetrahydropyranyloxy, each of which is optionally substituted by 1 to 3 substituents selected from a fluorine atom, a chlorine atom, a bromine atom, methyl, ethyl, methoxy and ethoxy, and $R^2$ is pyridyl or tetrahydropyranyl.

(3) The agent for use in the treatment and/or prophylaxis of an anxiety disorder, comprising the thiazole derivative or the pharmaceutically acceptable salt thereof of (1), wherein $R^1$ is pyridyl or pyrimidinyl, each of which is optionally substituted by 1 to 3 substituents selected from the group consisting of halogen; lower alkyl optionally substituted by lower alkoxy or morpholino; lower alkoxy; lower alkanoyl; and vinyl.

(4) The agent for use in the treatment and/or prophylaxis of an anxiety disorder, comprising the thiazole derivative or the pharmaceutically acceptable salt thereof of any one of (1) - (3), wherein $R^2$ is pyridyl.

(5) The agent for use in the treatment and/or prophylaxis of an anxiety disorder, comprising the thiazole derivative or the pharmaceutically acceptable salt thereof of any one of (1) - (3), wherein $R^2$ is tetrahydropyranyl.

(6) The agent for use in the treatment and/or prophylaxis of an anxiety disorder, comprising the thiazole derivative or the pharmaceutically acceptable salt thereof of (1), wherein the thiazole derivative represented by the formula (I) is a compound represented by any one of the following formulas (IA) - (IAA).

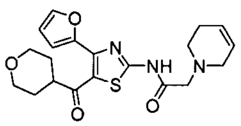

( IA )

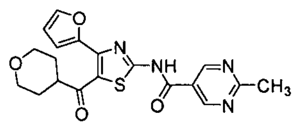

( IB )

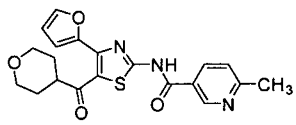

( IC )

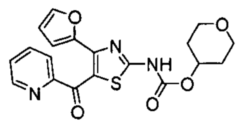

( ID )

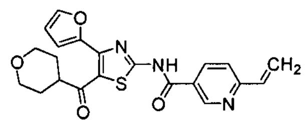

( IE )

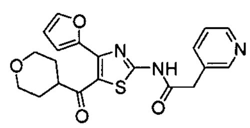

( IF )

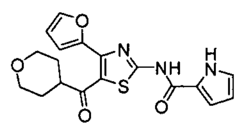

( IG )

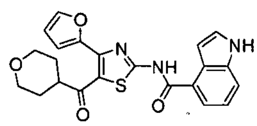

( IH )

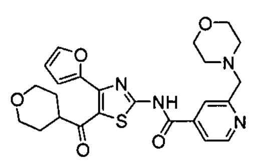

( II )

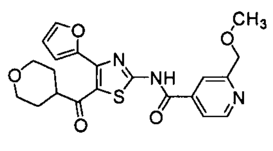

( IJ )

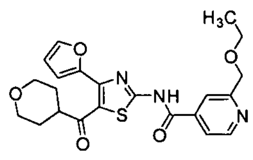

( IK )

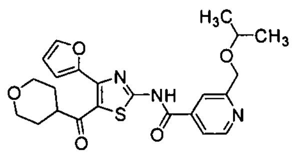

( IL )

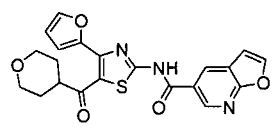
( IM )

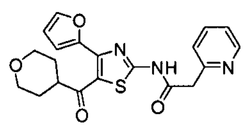
( IN )

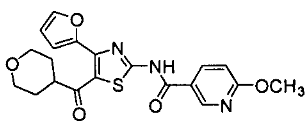
( IO )

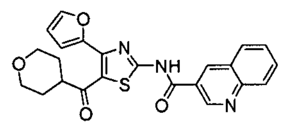
( IP )

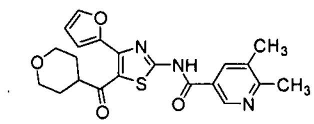
( IQ )

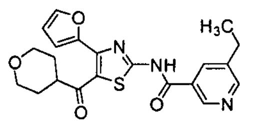
( IR )

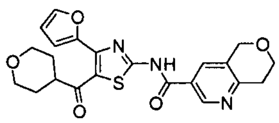
( IS )

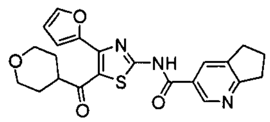
( IT )

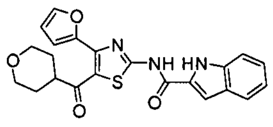
( IU )

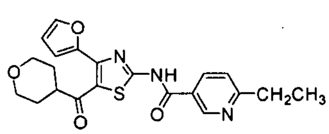
( IV )

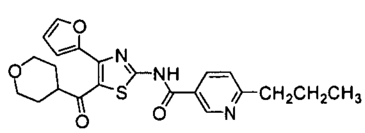
( IW )

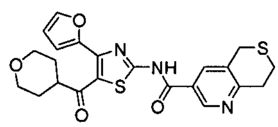
( IX )

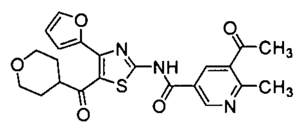
( IY )

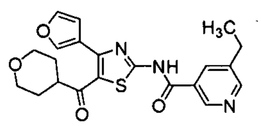
( IZ )

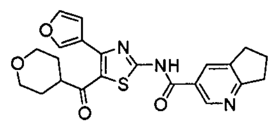
( IAA )

(7) The agent for use in the treatment and/or prophylaxis of an anxiety disorder, comprising the thiazole derivative or the pharmaceutically acceptable salt thereof of (1), wherein the thiazole derivative represented by the formula (I) is a compound represented by any one of the following formulas (IA) - (ID).

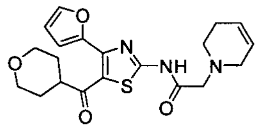
( IA )

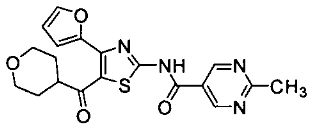
( IB )

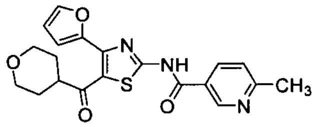
( IC )

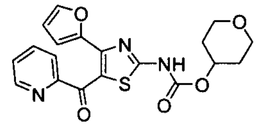
( ID )

(8) The agent of any of (1) - (7), wherein the anxiety disorder is panic disorder, agoraphobia, obsessive-compulsive disorder, social phobia, posttraumatic stress disorder, particular phobia or generalized anxiety disorder.

The present invention provides

1. A thiazole derivative represented by the formula (IC)

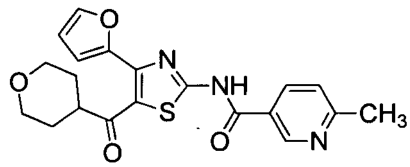

( IC )

or a pharmaceutically acceptable salt thereof for use in the treatment and/or prophylaxis of an anxiety disorder.

2. The thiazole derivative or the pharmaceutically acceptable salt thereof according to 1, for use in the treatment

and/or prophylaxis of the anxiety disorder selected from panic disorder, agoraphobia, obsessive-compulsive disorder, social phobia, posttraumatic stress disorder, particular phobia and generalized anxiety disorder.

3. Use of the thiazole derivative described in 1 or the pharmaceutically acceptable salt thereof, for the production of an agent for the treatment and/or prophylaxis of an anxiety disorder.

4. The use according to 3, wherein the anxiety disorder is panic disorder, agoraphobia, obsessive-compulsive disorder, social phobia, posttraumatic stress disorder, particular phobia or generalized anxiety disorder.

Effect of the Invention

The present invention provides an agent for use in the treatment and/or prophylaxis of an anxiety disorder (for example, panic disorder, agoraphobia, obsessive-compulsive disorder, social phobia, posttraumatic stress disorder, particular phobia, generalized anxiety disorder or the like) which comprises a thiazole derivative or a pharmaceutically acceptable salt thereof as an active ingredient.

Mode for Carrying Out the Invention

In the following, the compound represented by the formula (I) is sometimes referred to as compound (I). The compounds having other formula numbers are also referred to in the same manner.

The definition of each group in the formula (I) is as follows.

Examples of the lower alkyl moiety of the lower alkyl, the lower alkoxy and the lower alkanoyl include straight or branched alkyl having 1 to 10 carbon atoms, and more specific examples thereof include methyl, ethyl, propyl, isopropyl, butyl, isobutyl, sec-butyl, tert-butyl, pentyl, isopentyl, neopentyl, hexyl, heptyl, octyl, nonyl, decyl and the like.

Examples of the aralkyl include aralkyl having 7 to 16 carbon atoms, and more specific examples thereof include benzyl, phenethyl, phenylpropyl, phenylbutyl, phenylpentyl, phenylhexyl, phenylheptyl, phenyloctyl, phenylnonyl, phenyldecyl, naphthylmethyl, naphthylethyl, naphthylpropyl, naphthylbutyl, naphthylpentyl, naphthylhexyl, anthrylmethyl, anthrylethyl and the like.

Examples of the aryl include aryl having 6 to 14 carbon atoms, and more specific examples thereof include phenyl, naphthyl, azulenyl, anthryl and the like.

Examples of the aromatic heterocyclic group include a 5-membered or 6-membered monocyclic aromatic heterocyclic group containing at least one atom selected from a nitrogen atom, an oxygen atom and a sulfur atom, a bicyclic or tricyclic fused aromatic heterocyclic group in which 3 to 8-membered rings are fused, having at least one atom selected from a nitrogen atom, an oxygen atom, and a sulfur atom, and the like. More specific examples thereof include furyl, thienyl, pyrrolyl, imidazolyl, pyrazolyl, oxazolyl, isoxazolyl, oxadiazolyl, thiazolyl, isothiazolyl, thiadiazolyl, triazolyl, tetrazolyl, pyridyl, pyridazinyl, pyrimidinyl, pyrazinyl, triazinyl, benzofuranyl, benzothiophenyl, benzoxazolyl, benzothiazolyl, isoindolyl, indolyl, indazolyl, benzimidazolyl, benzotriazolyl, oxazolopyrimidinyl, thiazolopyrimidinyl, pyrrolopyridinyl, pyrrolopyrimidinyl, imidazopyridinyl, purinyl, quinolinyl, isoquinolinyl, cinnolinyl, phthalazinyl, quinazolinyl, quinoxalinyl, naphthyridinyl, furo[2,3-b]pyridyl, 6,7-dihydro-5H-cyclopenta[b]pyridyl, 7,8-dihydro-5H-pyrano[4,3-b]pyridyl, 7,8-dihydro-5H-thiopyrano[4,3-b]pyridyl and the like.

Examples of the aromatic heterocyclyl-alkyl include a group wherein an aromatic heterocyclic group is bonded to alkylene. The aromatic heterocyclic group include those exemplified in the above-mentioned aromatic heterocyclic group, and examples of the alkylene include alkylene having 1 to 10 carbon atoms, and specific examples thereof include methylene, ethylene, trimethylene, propylene, tetramethylene, pentamethylene, hexamethylene, heptamethylene, octamethylene, nonamethylene, decamethylene and the like. Specific examples of the aromatic heterocyclyl-alkyl include pyrrolylmethyl, pyrrolylethyl, thiazolylmethyl, pyridylmethyl, pyridylethyl, pyrimidinylmethyl, pyrimidinylethyl, indolylmethyl, benzimidazolylmethyl and the like.

Examples of the aliphatic heterocyclyl-alkyl include a group wherein the aliphatic heterocyclic group is bonded to alkylene. Examples of the aliphatic heterocyclic group include a 5-membered or 6-membered monocyclic aliphatic heterocyclic group containing at least one atom selected from a nitrogen atom, an oxygen atom and a sulfur atom, a bicyclic or tricyclic fused aliphatic heterocyclic group in which 3 to 8-membered rings are fused, having at least one atom selected from a nitrogen atom, an oxygen atom, and a sulfur atom, and the like. More specific examples thereof include aziridinyl, azetidinyl, pyrrolidinyl, piperidino, piperidinyl, azepanyl, 1,2,5,6-tetrahydropyridyl, imidazolidinyl, pyrazolidinyl, piperazinyl, homopiperazinyl, pyrazolinyl, oxiranyl, tetrahydrofuranyl, tetrahydro-2H-pyranyl, 5,6-dihydro-2H-pyranyl, 5,6-dihydro-2H-pyridyl, oxazolidinyl, morpholino, morpholinyl, thioxazolidinyl, thiomorpholinyl, 2H-oxazolyl, 2H-thioxazolyl, dihydroindolyl, dihydroisoindolyl, dihydrobenzofuranyl, benzimidazolidinyl, dihydrobenzoxazolyl, dihydrobenzothioxazolyl, benzodioxolinyl, tetrahydroquinolyl, tetrahydroisoquinolyl, dihydro-2H-chromanyl, dihydro-1H-chromanyl, dihydro-2H-thiochromanyl, dihydro-1H-thiochromanyl, tetrahydroquinoxalinyl, tetrahydroquinazolinyl, dihydrobenzodioxanyl and the like. Examples of the alkylene include alkylene having 1 to 10 carbon atoms, and specific examples thereof include methylene, ethylene, trimethylene, propylene, tetramethylene, pentamethylene, hexamethylene, heptamethylene, octamethylene, nonamethylene, decamethylene and the like. Specific examples of the aliphatic heterocyclyl-alkyl include 5,6-dihydro-2H-pyridylmethyl, 5,6-dihydro-2H-pyridylethyl, tetrahydro-2H-pyranylmethyl, 5,6-dihydro-2H-pyranylmethyl, 5,6-dihydro-2H-pyranylethyl, morpholinomethyl, morpholinoethyl, piperazinylmethyl, oxazolidinylmethyl and the like.

The halogen means each atom of fluorine, chlorine, bromine and iodine.

Compound (I) or a pharmaceutically acceptable salt thereof used in the present invention is preferably a compound having a potent antagonistic activity against adenosine $A_{2A}$ receptors from among various subtypes of adenosine receptors (e.g., adenosine $A_1$, $A_{2A}$, $A_{2B}$ and $A_3$ receptors).

Accordingly, compound (I) or a pharmaceutically acceptable salt thereof in the present invention is preferably a compound having a strong affinity for the adenosine $A_{2A}$ receptors. For example, the compound is preferably one having an inhibitory activity of 50% or more at a test compound concentration of $3\times10^{-8}$ mol/L, more preferably one having an inhibitory activity of 50% or more at a test compound concentration of $1\times10^{-8}$ mol/L, still more preferably one having an inhibitory activity of 50% or more at a test compound concentration of $3\times10^{-9}$ mol/L, further preferably one having an inhibitory activity of 50% or more at a test compound concentration of $1\times10^{-9}$ mol/L, in the adenosine $A_{2A}$ receptor binding test shown in the below-mentioned Test Example 1. In addition, the compound is preferably one having an inhibitory activity of 30 nmol/L or less in an inhibitory constant (Ki value) obtained by the test, more preferably one having an inhibitory activity of 10 nmol/L or less, still more preferably one having an inhibitory activity of 3 nmol/L or less, further preferably one having an inhibitory activity of 1 nmol/L or less.

Further, compound (I) or a pharmaceutically acceptable salt thereof used in the present invention is preferably a compound having selective affinity for the adenosine $A_{2A}$ receptors from among various subtypes of the adenosine receptors. For example, a compound having a higher affinity for the adenosine $A_{2A}$ receptors than that for the adenosine $A_1$ receptors is preferable. Specifically, for example, the compound is preferably a compound having 5 times or more affinity, more preferably 10 times or more affinity, further preferably 50 times or more affinity, even more preferably 100 times or more affinity, most preferably 500 times or more affinity for the adenosine $A_{2A}$ receptors as compared to that for the adenosine $A_1$ receptors (e.g., compared at Ki value).

The affinity can be determined according to a conventional method, for example, according to the method of Test Example 1 to be mentioned below, or the methods described in a document [for example, Naunyn Schmiedebergs Arch Pharmacol., 355(1), p. 59 (1987); Naunyn Schmiedebergs Arch Pharmacol. 355(2), p. 204 (1987); Br. J. Pharmacol. 117(8), p. 1645 (1996) and the like].

More specifically, compound (I) is preferably a compound wherein $R^1$ is phenyl optionally substituted by 1 to 3 substituents selected from halogen, $C_{1-6}$ alkyl optionally substituted by $C_{1-6}$ alkoxy or morpholino, $C_{1-6}$ alkanoyl, vinyl and $C_{1-6}$ alkoxy; pyridyl optionally substituted by 1 to 3 substituents selected from halogen, $C_{1-6}$ alkyl optionally substituted by $C_{1-6}$ alkoxy or morpholino, $C_{1-6}$ alkanoyl, vinyl and $C_{1-6}$ alkoxy; pyrimidinyl optionally substituted by 1 to 3 substituents selected from halogen, $C_{1-6}$ alkyl optionally substituted by $C_{1-6}$ alkoxy or morpholino, $C_{1-6}$ alkanoyl, vinyl and $C_{1-6}$ alkoxy; 5,6-dihydro-2H-pyridylmethyl optionally substituted by 1 to 3 substituents selected from halogen, $C_{1-6}$ alkyl and $C_{1-6}$ alkoxy; 2,3,4,5-tetrahydropyranyloxy; pyrrolyl; indolyl; oxazolopyridyl; quinolyl; 1H-3,4-dihydropyranopyridinyl; 1H-3,4-dihydrothiopyranopyridinyl; cyclopentapyridyl; or pyridylmethyl,

more preferably a compound wherein $R^1$ is phenyl optionally substituted by 1 to 3 substituents selected from a fluorine atom, a chlorine atom, methyl and methoxy; pyridyl optionally substituted by 1 to 3 substituents selected from a fluorine atom, a chlorine atom, methyl and methoxy; pyrimidinyl optionally substituted by 1 to 3 substituents selected from a fluorine atom, a chlorine atom, methyl and methoxy; 5,6-dihydro-2H-pyridylmethyl optionally substituted by 1 to 3 substituents selected from a fluorine atom, a chlorine atom, methyl and methoxy; or 2,3,4,5-tetrahydropyranyloxy,

still more preferably a compound wherein $R^1$ is pyridyl substituted by 1 to 3 substituents selected from a chlorine atom, methyl and methoxy; pyrimidinyl substituted by 1 to 3 substituents selected from chlorine atom, methyl and methoxy; 5,6-dihydro-2H-pyridylmethyl; or 2,3,4,5-tetrahydropyranyloxy. More specifically, compound (I) is preferably, for example, compounds of the following formulas (IA) - (IAA).

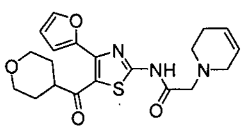
( IA )

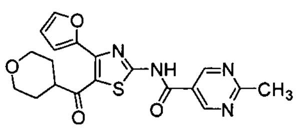
( IB )

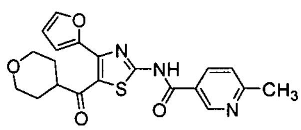
( IC )*

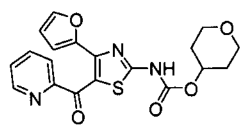
( ID )

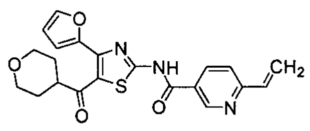

( IE )

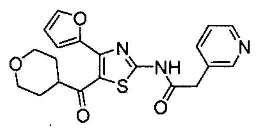

( IF )

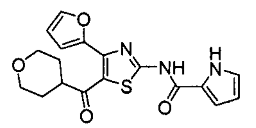

( IG )

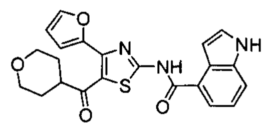

( IH )

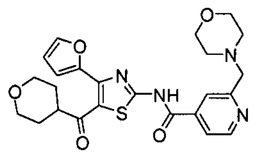

( II )

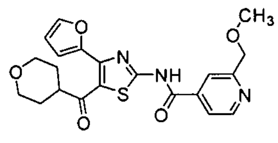

( IJ )

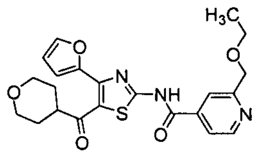

( IK )

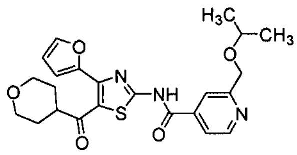

( IL )

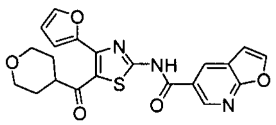

( IM )

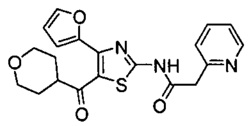

( IN )

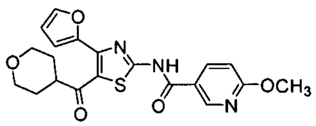

( IO )

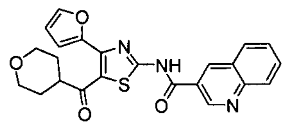

( IP )

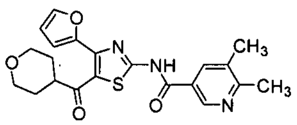

( IQ )

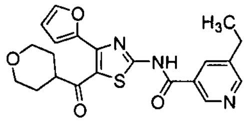

( IR )

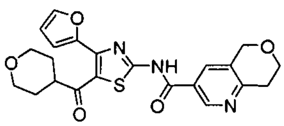

( IS )

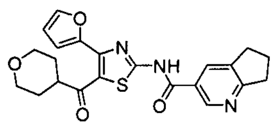

( IT )

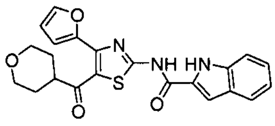

( IU )

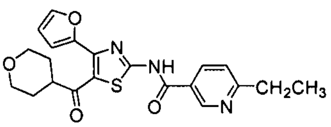

( IV )

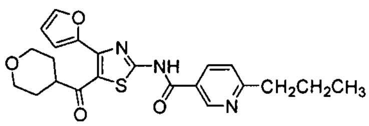

( IW )

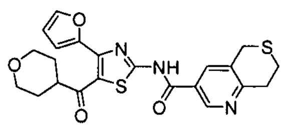

( IX )

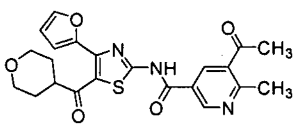

( IY )

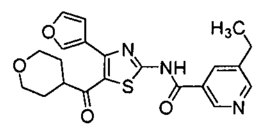

( IZ )

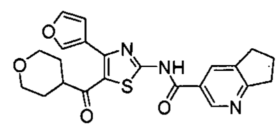

( IAA )

** Compound of the invention*

The pharmaceutically acceptable salts of compound (I) include, for example, pharmaceutically acceptable acid addition salts, metal salts, ammonium salts, organic amine addition salts, amino acid addition salts, and the like. The pharmaceutically acceptable acid addition salts of compound (I) include, for example, inorganic acid salts such as hydrochloride, hydrobromate, nitrate, sulfate, and phosphate; organic acid salts such as acetate, oxalate, maleate, fumarate, citrate, benzoate, and methane sulfonate, and the like. Examples of the pharmaceutically acceptable metal salts include alkali metal salts such as a sodium salt, and a potassium salt; alkaline earth metal salts such as a magnesium salt, and a calcium salt; an aluminum salt; a zinc salt, and the like. Examples of the pharmaceutically acceptable ammonium salts include salts of ammonium, tetramethylammonium, and the like. Examples of the pharmaceutically acceptable organic amine addition salts include addition salts of morpholine, piperidine, or the like. Examples of the pharmaceutically acceptable amino acid addition salts include addition salts of lysine, glycine, phenylalanine, aspartic acid, glutamic acid, or the like.

Compound (I) can be produced according to a known method, for example, the method described in WO 2005/063743 and the like.

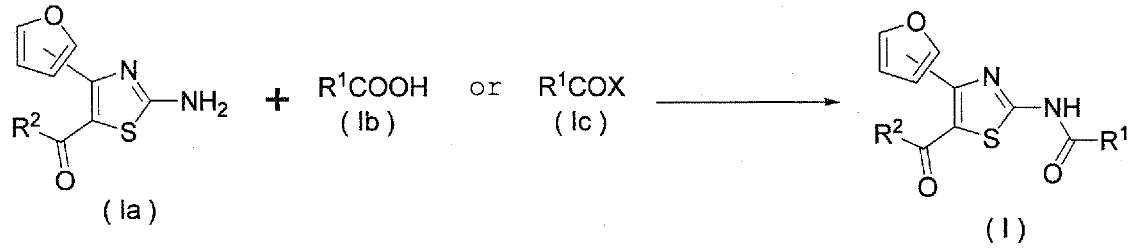

wherein $R^1$ and $R^2$ are as defined above, and X represents a chlorine atom, a bromine atom or the like.

Specifically, as shown in the above-mentioned formula, compound (I) can be produced, for example, by reacting compound (Ia) described in WO 2005/063743 with preferably 0.5 to 5 equivalents of compound (Ib) in a solvent such as methanol, dichloromethane, chloroform, toluene, ethyl acetate, acetonitrile, tetrahydrofuran (THF), N,N-dimethylformamide (DMF), N,N-dimethylacetamide (DMA), pyridine, water, or a mixed solvent thereof and the like, preferably in the presence of 1 to 5 equivalents of a condensing agent such as 1,3-dicyclohexanecarbodiimide (DCC), 1-ethyl-3-(3-dimethylaminopropyl)carbodiimide (EDC) hydrochloride and the like, if necessary, in the presence of preferably 1 to 5 equivalents of 1-hydroxybenzotriazole (HOBt) monohydrate, 4-dimethylaminopyridine (DMAP) and the like, at a temperature between -20°C and the boiling point of the solvent used, for 5 min to 72 hr.

Alternatively, compound (I) can also be produced, for example, by reacting compound (Ia) described in WO 2005/063743 with preferably 1 to 10 equivalents of compound (Ic) without solvent or in a solvent such as dichloromethane, chloroform, 1,2-dichloroethane, toluene, ethyl acetate, acetonitrile, THF, DMF, DMA, pyridine and the like, if necessary, in the presence of preferably 1 to 10 equivalents of a base such as potassium carbonate, triethylamine, 4-dimethylaminopyridine (DMAP) and the like, at a temperature between -20°C and 150°C, for 5 min to 72 hr.

Compound (I) may exist as stereoisomers such as geometrical isomers or optical isomers, or tautomers. Any possible isomers and a mixture thereof, including those mentioned above, can be used for the agent of the present invention for the treatment and/or prophylaxis of an anxiety disorder.

To obtain a salt of compound (I), when the compound (I) is obtained in the form of a salt, it may be purified as it is. Further, when the compound is obtained in a free form, compound (I) may be dissolved or suspended in a suitable solvent, followed by addition of an acid or a base to form a salt. Then, the resulting salt may be isolated and purified.

The compound (I) or a pharmaceutically acceptable salt thereof may exist in the form of an adduct with water or various solvents. Such adduct can also be used for the agent of the present invention for the treatment and/or prophylaxis of an anxiety disorder.

A pharmacological action of the representative compound (I) is now specifically explained by way of Experimental Examples.

Test Example 1: Adenosine Receptor Binding Action

(1) Adenosine $A_{2A}$ Receptor Binding Test

The test can be performed according to, for example, the method of Varani et al. (British Journal of Pharmacology, 117, p. 1693 (1996)).

Specifically, for example, human recombinant Adenosine $A_{2A}$ receptors are expressed in HEK-293 cells. The cell membranes of the receptor-expressing cells are collected, and a cell membrane suspension is prepared. After dilution with tris(hydroxymethyl)-aminomethane hydrochloride (Tris HCl) buffer, tritium-labeled 2-[*p*-(2-carboxyethyl)phenethylamino]-5'-(*N*-ethylcarboxamido)adenosine ($^3$H-CGS21680: 50 mmol/L) and a test compound solution (dimethyl sulfoxide solution of the test compound) are added to the cell membrane suspension for binding to the receptors. After the reaction, the mixture is subjected to rapid suction filtration using glass-fiber filter paper, and the radioactivity of the glass-fiber filter paper is measured. In this way, the inhibitory rate of the test compound for the human adenosine $A_{2A}$ receptor binding ($^3$H-CGS21680 binding) can be determined.

The test can also be performed according to the method of Bruns et al. (Molecular Pharmacology, Vol. 29, p. 331, 1986).

Specifically, for example, rat striatum is suspended in 50 mL of ice-cooled Tris HCl buffer (50 mmol/L, pH 7.7) using a Polytron homogenizer and the suspension is centrifuged. The resulting precipitate is resuspended by adding Tris HCl buffer (50 mmol/L) thereto, followed by centrifugation in the same manner. The resulting final precipitate is suspended in Tris HCl buffer (50 mmol/L) [containing magnesium chloride (10 mmol/L), and adenosine deaminase (0.02 units/mg tissue)] to prepare the suspension at the tissue concentration of 5 mg (wet weight)/mL. Tritium-labeled CGS-21680 (final concentration of 6.0 mmol/L), and the test compound solution (dimethyl sulfoxide solution of test compound diluted with Tris HCl buffer) are added. The mixture is allowed to stand at 25°C for 120 minutes, followed by rapid suction

filtration using glass-fiber filter paper, and then immediately washed with ice-cooled Tris HCl buffer (50 mmol/L). The glass-fiber filter paper is then placed in a vial, and MicroScinti (PKI) is added. Then, the radioactivity is measured with a TopCount (PerkinElmer), whereby the inhibitory rate for rat adenosine $A_{2A}$ receptor binding ($^3$H-CGS21680 binding) of the test compound can be determined.

The inhibitory rate can be calculated by the following equation.

[Equation 1]

$$\text{Inhibitory rate}(\%) = \left(1 - \frac{\text{Amount of binding in the presence of drug} - \text{Amount of non-specific binding}}{\text{Total amount of binding} - \text{Amount of non-specific binding}}\right) \times 100$$

In the equation, the total amount of binding refers to the bound radioactivity of $^3$H-CGS21680 in the absence of the test compound. The amount of non-specific binding refers to the bound radioactivity of $^3$H-CGS21680 in the presence of 50 μmol/L of 5'-N-ethylcarboxamideadenosine (NECA) or 100 μmol/L of cyclopentyladenosine (CPA). The amount of binding in the presence of drug refers to the bound radioactivity of $^3$H-CGS21680 in the presence of the test compound.

In the above test, the inhibitory rate for the adenosine $A_{2A}$ receptor at different concentrations of the test compound or a pharmaceutically acceptable salt thereof, and the test compound concentration at which the test compound inhibits binding by 50% ($IC_{50}$) can be calculated by appropriately adjusting the concentration of the test compound.

The inhibition constant (Ki value) of the test compound for the adenosine $A_{2A}$ receptor binding can be calculated according to the following equation.

[Equation 2]

$$Ki = IC_{50}/(1 + L/Kd)$$

In the equation, L denotes the concentration of the $^3$H-CGS21680 used in the test, and Kd is the dissociation constant of the $^3$H-CGS21680 used in the test.

Instead of $^3$H-CGS21680, $^3$H-5-amino-7-(2-phenylethyl)-2-(2-furyl)pyrazolo[4,3-*e*]-1,2,4-triazolo[1,5-*c*]pyrimidine ($^3$H-SCH58261) and the like may be used.

(2) Adenosine $A_1$ Receptor Binding Test

The inhibition constant (Ki value) of the test compound for the adenosine $A_1$ receptors can be calculated in the same manner as in (1), using the materials below.

Specifically, for example, human $A_1$ receptor-expressing CHO cell membranes are used, and, as the labeled compound, for example, tritium-labeled 1,3-dipropyl-8-cyclopentylxanthine ($^3$H-DPCPX) is used. The amount of non-specific binding can be determined by measuring the $^3$H-DPCPX bound radioactivity in the presence of, for example, 100 μmol/L of (-)-$N^6$-2-phenylisopropyl adenosine (R(-)-PIA). The affinity of the test compound for the human adenosine $A_1$ receptors can be confirmed in this manner.

Alternatively, for example, rat $A_1$ receptor-expressing cell membrane (PerkinElmer) is used, and as the labeled compound, for example, tritium-labeled $N^6$-cyclohexyladenosine ($^3$H-CHA) is used. For the measurement of the amount of non-specific binding, $^3$H-CHA bound radioactivity is measured in the presence of, for example, 10 μmol/L of DPCPX, and the affinity of the test compound for the rat adenosine $A_1$ receptors can be confirmed.

By the foregoing tests (1) and (2), the selective affinities of the thiazole derivative or a pharmaceutically acceptable salt thereof used in the present invention for the adenosine $A_{2A}$ receptors can be confirmed.

(3) Affinity of compound (I) or a pharmaceutically acceptable salt thereof for adenosine receptors

Some of the examples of the affinities of compound (I) or a pharmaceutically acceptable salt thereof for the adenosine $A_1$ receptor and the adenosine $A_{2A}$ receptor are presented below. Note that the test results below are those measured by MDS Pharma Services Inc. according to the foregoing methods.

[Table 1] The affinity for adenosine receptor

| Compound No. | inhibitory rate* for human adenosine $A_{2A}$ receptor binding ($^3$H-CGS21680 binding) | inhibitory rate* for human adenosine $A_1$ receptor binding ($^3$H-DPCPX binding) |
|---|---|---|
| (IA) | 92% | 14% |
| (IB) | 98% | 4% |
| (IC)** | 88% | 29% |
| (ID) | 100% | 28% |
| * inhibitory rate for compound 100 nmol/L<br>** Compound of the invention | | |

The above-mentioned test has confirmed that compound (I) shows selective affinity for the adenosine $A_{2A}$ receptors.

Test Example 2 Adenosine Receptor Binding Activity (2)

In the same manner as in the above-mentioned Test Example 1, the affinity of compound (IE) - (IAA) for adenosine receptors was confirmed (test results were those measured by Ricerca Biosciences, LLC according to the foregoing methods).

[Table 2] The affinity for adenosine receptor

| Compound No. | inhibitory rate* for human adenosine $A_{2A}$ receptor binding ($^3$H-CGS21680 binding) | inhibitory rate* for human adenosine $A_1$ receptor binding ($^3$H-DPCPX binding) | Compound No. | inhibitory rate* for human adenosine $A_{2A}$ receptor binding ($^3$H-CGS21680 binding) | inhibitory rate* for human adenosine $A_1$ receptor binding ($^3$H-DPCPX binding) |
|---|---|---|---|---|---|
| (IE) | 93% | 33% | (IF) | 107% | 50% |
| (IG) | 102% | 91% | (IH) | 98% | 67% |
| (II) | 85% | 19% | (IJ) | 93% | 21% |
| (IK) | 92% | 24% | (IL) | 85% | 20% |
| (IM) | 98% | 47% | (IN) | 93% | 21% |
| (IO) | 97% | 56% | (IP) | 98% | 18% |
| (IQ) | 100% | 18% | (IR) | 107% | 30% |
| (IS) | 90% | 10% | (IT) | 91% | 37% |
| (IU) | 110% | 36% | (IV) | 98% | 23% |
| (IW) | 98% | 23% | (IX) | 101% | 18% |
| (IY) | 97% | 8% | (IZ) | 102% | 21% |
| (IAA) | 98% | 9% | | | |
| * inhibitory rate for compound 100 nmol/L | | | | | |

From the above tests, it has been confirmed that compound (I) shows selective affinity for the adenosine $A_{2A}$ receptors.

Test Example 3: Effect of compound (I) or a pharmaceutically acceptable salt thereof on marble burying behavior test in mice

This test is known as one of the test systems for anxiolytics (Folia Pharmacologica Japonica, 126, p.94 (2005)).

Marble-burying behavior is a behavior of the mouse to bury marbles in spread planer bedding on which the marbles were placed, and is suppressed, without being accompanied by motor inhibition, by selective serotonin reuptake inhibitors (SSRIs). Since the behavior of the mouse to attempt to cover and bury harmless marbles in planer bedding is apparently similar to compulsions in patients with obsessive-compulsive disorders that are repeated while they recognize them as being unreasonable, and also since SSRIs are effective as therapeutic drugs for obsessive-compulsive disorders, and for other reasons, the marble burying behavior is getting positioned as an animal model of obsessive-compulsive disorder

The male ICR mice (weighing 27.6 to 42.9 g; Japan SLC, Inc.) were used in this study. One hour after oral administration of a vehicle [water for injection (Otsuka Pharmaceutical Factory) containing methylcellulose (MC) at a concentration of 0.5 w/v%; 0.5 w/v% MC solution; control group] or the test compound (prepared by being suspended in 0.5 w/v% MC solution to obtain a dosing concentration of 0.1 mL per 10 g of mouse body weight; drug group), each mouse was placed in a cage, in which 25 marbles (18 mm in diameter) were equally placed on the surface of planer (up to 5 cm from the bottom of the cage). At 30 minutes later, the number of marbles more than half-buried in the planer was counted. The counting was performed under blinding conditions.

The effects of the drug are expressed as the actually measured number of buried marbles and the inhibition rate calculated by the following equation.

[Equation 3]

$$\text{Inhibition (\%)} = \frac{\left(\begin{array}{l}\text{number of buried}\\ \text{marbles in}\\ \text{control group}\end{array}\right) - \left(\begin{array}{l}\text{number of}\\ \text{buried marbles}\\ \text{in drug group}\end{array}\right)}{\left(\begin{array}{c}\text{number of buried marbles in}\\ \text{control group}\end{array}\right)} \times 100$$

When the compound (IC) was administered at a dose of 3 mg/kg, the mouse marble burying behavior was significantly inhibited (% inhibition: 73.7%).

Since the compound (IC) exhibited an inhibition effect in the above-described test, the compound (I) having a selective affinity for adenosine $A_{2A}$ receptors was considered to be useful in the treatment and/or prophylaxis of anxiety disorders, including obsessive-compulsive disorders.

Test Example 4: Action of compound (I) or a pharmaceutically acceptable salt thereof in rat social interaction test

Two separately reared rats weighing nearly the same are placed in the same measuring cage and examined for social interactions such as sniffing, following, and grooming exhibited by the two rats. It is known that these social interactions increase with administration of existing anxiolytics. This test is thought as an animal model of social phobia or generalized anxiety disorders because it is consider that contradictory social environments between two rats cause stress (Eur. J. Pharmacol., 408, p.41 (2000)).

The male SD rats (weighing 201.8 to 285.6 g; Charles River Japan Inc.)were used in this study. On the day of behavioral evaluation, the animals were acclimatized to the test environment from 4 hours before the start of the experiment. Sixty minutes after oral administration of a vehicle (0.5 w/v% MC solution) or a test compound (prepared in suspension in 0.5 w/v% MC solution to obtain a dosing concentration of 0.5 mL per 100 g of rat body weight), two rats were allowed to meet each other in an acrylic box (50 x 50 x 50 cm). The total time of social interaction behaviors (sniffing, following, grooming and the like) during monitoring for 10 minutes immediately after meeting (between-rats interaction time) were measured with a stopwatch.

When the compound (IC) was administered at a dose of 0.3 mg/kg, the between-rats interaction time increased significantly (119.3 $\pm$9.0 seconds versus 85.2 $\pm$7.6 seconds for vehicle treatment, P=0.01545, Dunnett test). Spontaneous activity also increased significantly (12922.7 $\pm$646.8 counts versus 8635.3 $\pm$506.4 counts for vehicle treatment, $P<0.001$, Dunnett test).

Since the compound (IC) exhibited an effect in the above-described test, the compound (I) having a selective affinity for adenosine $A_{2A}$ receptors was considered to be useful in the treatment and/or prevention of anxiety disorders, including social phobia and/or generalized anxiety disorders.

Test Example 5: Effect of compound (I) or a pharmaceutically acceptable salt thereof on drug-induced anxiety-like behavior in rat social interaction test

Using the method of Test Example 4, the effect of a compound on the anxiety induced with administration of an anxiogenic substance was examined. Yohimbine (adrenalin $\alpha 2$ receptor antagonist) is known as one of the anxiogenic

substance. It is thought that locus coeruleus hyperactivity that accompanies $\alpha 2$ receptor antagonizing action is contributory to the anxiety-inducing action of Yohimbine. Anxiety symptoms that develop with administration of Yohimbine to healthy persons were reported to be similar to panic disorders, in the pathophysiology of which abnormal locus coeruleus activity is considered to be largely involved. For this reason, Yohimbine-induced anxiety is thought to be a model well reflecting anxiety symptoms (for example, panic disorders) that accompany abnormal locus coeruleus activity.

Meanwhile, meta-chlorophenylpiperadine hydrochloride (mCPP) is an anxiogenic substance having a 5-$HT_2$ receptor stimulation action. It was reported that mCPP induced anxiety symptoms in healthy subjects and aggravated the symptoms of generalized anxiety disorder in humans. The mCPP-induced anxiety response is an experimental model that well reflects anxiety (for example, generalized anxiety disorder) caused by abnormal signaling especially via 5-$HT_2$ receptors.

First, the effect on Yohimbine-induced anxiety was examined.

In a group receiving a vehicle (0.5 w/v% MC solution) administered 30 minutes before the measurement, the observed mean between-rats interaction time was 90.6$\pm$7.1 seconds. In contrast, in a group receiving Yohimbine administered 30 minutes before the measurement (prepared by being dissolved in 0.5 w/v% MC solution to obtain a dosing concentration of 0.25 mL per 100 g of rat body weight, and orally administered at a dose of 5 mg/kg), the between-rats interaction time decreased significantly (57.6$\pm$3.4 seconds, P=0.0010, Aspin-Welch test). When the compound (IC) (prepared in suspension in 0.5 w/v% MC solution to obtain a dosing concentration of 0.25 mL per 100 g of rat body weight) was administered at a dose of 0.03 mg/kg 30 minutes before administration of Yohimbine, the above-described interaction time reduction by Yohimbine (5 mg/kg) improved significantly (0.03 mg/kg: 90.3$\pm$5.0 seconds, P = 0.00019, Steel test).

Next, the effect on mCPP-induced anxiety was examined.

In a group receiving a vehicle (0.5 w/v% MC, orally administered at 0.25 mL per 100 g of rat body weight) administered 30 minutes before the measurement, the observed mean between-rats interaction time was 83.4$\pm$5.1 seconds. In contrast, in a group receiving mCPP administered 30 minutes before the measurement (prepared by being dissolved in 0.5 w/v% MC solution to obtain a dosing concentration of 0.25 mL per 100 g of rat body weight, and orally administered at a dose of 0.5 mg/kg), the between-rats interaction time decreased significantly (32.5$\pm$3.6 seconds, P<0.001, Student's t-test). When the compound (IC) (a suspension in 0.5 w/v% MC solution prepared to obtain a dosing concentration of 0.25 mL per 100 g of rat body weight) was administered at a dose of 0.3 mg/kg 30 minutes before administration of mCPP, the above-described interaction time reduction by mCPP (0.5 mg/kg) improved significantly (0.3 mg/kg: 62.4$\pm$6.9 seconds, P<0.001, Dunnett test).

Since the compound (IC) exhibited an effect in the above-described test, the compound (I) having a selective affinity for adenosine $A_{2A}$ receptors was considered to be useful in the treatment and/or prophylaxis of drug-induced anxiety disorders. Also, the compound (I) was considered to be useful in the treatment and/or prevention of anxiety disorders, including panic disorders and generalized anxiety disorders.

Test Example 6: Effect of compound (I) or a pharmaceutically acceptable salt thereof on Vogel conflict test in rats

This test is to measure drinking counts (number of shocks during drinking) when an electric shock is applied to a water-deprived rat at each time that the rat drinks water. Under this condition, a rat is thought to be placed under a state of conflict between the motivation to drink (positive reinforcing factor) and the aversion to receiving an electric shock (negative reinforcing factor, punishment). When anxiolytics are administered to a rat, anxiety about punishing stimuli is suppressed and behavior in an attempt to take a positive reinforcer is observed. This test is thought to be an animal model of generalized anxiety disorders (Folia Pharmacologica Japonica, 115, p.5 (2005), Japanese Journal of Clinical Psychopharmacology, 9, p.2389 (2006)).

The male Wistar rats (weighing 148.4 to 211.7 g; Japan SLC, Inc.) and an operant experimental apparatus were used in this study. The chamber consisted of a soundproof box, and a test cage (25 $\times$ 30 $\times$ 25 cm), a shock generator, a controller, a licking sensor and PC analysis system placed in the soundproof box. The test cage has 20 grids on the floor and a metal nozzle on the inner wall. Through the nozzle, rats were allowed for free access to drinking water. A mild electric current was applied between the floor grids and the metal nozzle in the test cage. Current conduction by licking (drinking) was detected with the licking senor and measured with the PC analysis system via the controller. An electric current was generated using the shock generator between the floor grids and the metal nozzle in the test cage to apply electric shocks to rats. The rats were acclimated in the test cage for 10 minutes, then transferred to the housing cage and deprived from water. After 24 hours elapsed, the animals were placed in the test cage, allowed for free access to drinking water for 5 minutes (without electric shocks), again transferred to the housing cage and deprived from water. After 24 hours elapsed, the animals were allowed for free access to drinking water for 5 minutes in the test cage (pre-drug session without electric shocks). Twenty nozzle licks were counted as one drinking response and drinking frequency (drinking count) was determined (pre-value). At 60 minutes after the pre-drug session, a vehicle (0.5 w/v% MC solution) or a test compound (a suspension in 0.5 w/v% MC solution prepared to obtain a dosing concentration of

0.5 mL per 100 g of rat body weight) was orally administered, 60 minutes after administration, the test was performed for 5 minutes (test-punished session, with electric shocks). In the test-punished session, an electric shock of 0.16 mA, 0.2 sec/shock was delivered to the animals between the floor grid and the drinking nozzle after every 20th lick by drinking of rat (punished drinking). Animals that did not drink water were excluded from the evaluation and the drinking counts only in animals that were in a state of conflict were averaged and compared.

In the test-punished session, the drinking count was decreased by electric shocks compared with that in the pre-drug session (pre-drug session: 26.7±1.2 times, vehicle treatment group: 6.1±0.8 times). When the compound (IC) was administered at a dose of 0.03 mg/kg, the number of electric shocks received increased significantly (12.7±1.7 times, P=0.003846, Steel-test).

Since the compound (IC) exhibited an effect in the above-described test, the compound (I) having a selective affinity for adenosine $A_{2A}$ receptors was considered to be useful in the treatment and/or prophylaxis of anxiety disorders, including generalized anxiety disorders.

Test Example 7: Effect of compound (I) or a pharmaceutically acceptable salt thereof on elevated plus maze test in rat

In this test, a conflict arises between rodent's exploratory drive and an innate aversion to bright open new spaces (open arms), accordingly, it has been reported that drugs having anti-anxiety activity increase the time spent in the open arms and the number of open arm entries, while anxiogenic substance decreases these parameters (Japanese Journal of Clinical Psychopharmacology, 9, p.2389 (2006)). This test is thought to be an animal model of generalized anxiety disorders (Folia Pharmacologica Japonica, 115, p.5 (2005), Japanese Journal of Clinical Psychopharmacology, 9, p.2389 (2006)).

The male SD rats (weighing 133.8 to 260.2 g; Charles River Japan Inc.) were used in this study. An apparatus was used wherein two open arms (50x10 cm) extending on the same line from a 10 cm-square central area and two enclosed arms (50x10 cm) surrounded by a wall of 40 cm height are perpendicularly crossing. Sixty minutes after oral administration of a vehicle (0.5 w/v% MC solution) or a test compound (a suspension in 0.5 w/v% MC solution prepared to obtain a dosing concentration of 0.5 mL per 100 g of rat body weight), each rat was placed in the center of the elevated plus maze with its head facing toward the enclosed arm and immediately observed for its behavior for 5 minutes. The rat behavior on the maze was recorded with a digital video camera positioned on the ceiling of the laboratory. The times spent and number of entries in the open arms, enclosed arms and central platform, and distance travelled were determined using analysis software. The time spent in the open arms, percentage of open arm entries (ratio of the number of entries in the open arms to the total number of entries in the open and enclosed arms) and total distance travelled on the maze during the monitoring period were determined to evaluate the influences of the test compound.

The time spent in the open arms, the percentage of open arm entries, and total distance travelled on the maze in rats with administration of the vehicle were 21.6 ±7.4 seconds, 15.5 ± 3.0%, and 2521.97±95.34 cm, respectively. When the compound (IC) was administered at a dose of 0.1 mg/kg, significantly increased the time spent in the open arms and the percentage of open arm entries (the time spent: 61.6±10.9 seconds, P=0.01687, the percentage: 33.4±6.3%, P=0.04759, Steel test). Compound (IC) did not affect the total distance travelled on the maze (2414.83±102.13 cm).

Since the compound (IC) exhibited an effect in the above-described test, the compound (I) having a selective affinity for adenosine $A_{2A}$ receptors was considered to be useful in the treatment and/or prophylaxis of anxiety disorders, including generalized anxiety disorders.

Test Example 8: Effect of compound (I) or a pharmaceutically acceptable salt thereof on the conditioned fear stress (CFS) test in rats

In this test (Folia Pharmacologica Japonica, 113, p.113 (1999)), the influences of psychological stress resulting from past aversion experience on living organisms can be measured as behavioral suppression (freezing reaction) or autonomic nervous system hyperactivity (increases in respiratory rate and blood pressure).

The male SD rats (weighing 140.2 to 320.0 g; Charles River Japan Inc.) and a contextual learning test system were used in this study. The experimental apparatus is consisted of a soundproof box, and a test cage (20 × 20 × 25 cm), a shock generator, a controller, an external monitor and PC analysis system, which were placed in the soundproof box. The test cage has stainless bars on the floor at 1-cm intervals. A scrambled electric current was generated from the shock generator using a command of the special program. The inside of the soundproof box was equipped with a speaker. A buzzer sound of 65 dB was generated via the controller using a command of the special program. The animal behavior within the test cage was recorded with a CCD camera, which was positioned on the ceiling of the soundproof box. Immobilization continuing for at least 2 seconds was defined as freezing. The percentage of freezing behavior was determined by (Freezing time/Test time) × 100. Conditioning trials were performed under the conditions shown below. Each rat was placed in the cage for 5 minutes and received electric shocks (0.3 mA × 5 seconds) from the floor grids

six times in total (60, 90, 120, 150, 180 and 210 seconds). A buzzer sound (10 kHz, 65 dB) was generated for 10 seconds from 5 seconds before each electric shock. Retention trials were performed under the conditions shown below. On the following day of conditioning, sixty minutes after the administration of a vehicle (0.5 w/v% MC solution) or a test compound (a suspension in 0.5 w/v% MC solution prepared to obtain a dosing concentration of 0.5 mL per 100 g of rat body weight), the rat was placed in the test cage for 5 minutes. According to the same schedule as that on the previous day, only a buzzer sound was generated six times and the duration of freezing behavior was measured. The 5-minute test period was divided into a pre-tone period (0 to 1 minute) from immediately after exposure to the test environment until generation of a buzzer sound, a with-tone period (1 to 4 minutes) during generation of a buzzer sound and a post-tone period (4 to 5 minutes) after generation of a buzzer sound. The percentage of freezing behavior during these periods was compared.

Administration of the compound (IC) at a dose of 0.3 mg/kg, significantly decreased the percentage of freezing behavior in the with-tone period (vehicle treatment group: 90.2±2.6 seconds, compound treatment group: 57.1±9.9 seconds, P=0.02867, Steel test). Also compound (IC) significantly decreased the percentage of freezing behavior also in the post-tone period (vehicle treatment group: 59.8±9.6 seconds, compound treatment group: 23.4±8.1 seconds, P=0.01559, Dunnett test).

Therefore, the compound (I) having a selective affinity for adenosine $A_{2A}$ receptors was considered to be useful in the treatment and/or prevention of anxiety disorders, including psychological stress-induced anxiety disorders (posttraumatic stress disorders).

While compound (IC) or a pharmaceutically acceptable salt thereof can be administered alone as it is, usually it is preferably provided in the form of various pharmaceutical preparations. Such pharmaceutical preparations can be used for animals and human.

The pharmaceutical preparation according to the present invention may contain, as the active ingredient, compound (IC) or a pharmaceutically acceptable salt thereof either alone or as a mixture with any other therapeutic active ingredient. Furthermore, these pharmaceutical preparations are prepared by mixing the active ingredient with one or more pharmaceutically acceptable carriers (e.g., diluents, solvents, excipients, or the like), and then subjecting the mixture to any method well-known in the technical field of pharmaceutics.

As for the administration route, it is preferable to select the most effective route of administration for treatment. Examples of the administration route include oral administration, and parenteral administration, for example, such as intravenous or transdermal administration and the like.

Examples of the dosage form include tablets, injections, external preparations, and the like.

Suitable dosage forms for the oral administration, for example, tablets, can be prepared by using excipients such as lactose, disintegrators such as starch, lubricants such as magnesium stearate, or binders such as hydroxypropylcellulose, or the like.

Suitable dosage forms for the parenteral administration, for example, injections, can be prepared by using diluents or solvents such as a saline solution, a glucose solution, or a mixture of brine and glucose solution, or the like.

A dosage form suitable for external preparation is not particularly limited and, for example, ointment, cream, liniment, lotion, cataplasm, plaster, tape and the like can be included. For example, ointment, cream and the like can be produced by, for example, dissolving or mixing-dispersing the active ingredient in a base such as white petrolatum and the like.

The dose and administration frequency of compound (IC) or a pharmaceutically acceptable salt thereof varies depending on administration form, age and body weight of patients, properties or severity of the symptoms to be treated and the like. For general oral administration, 0.001 - 1000 mg, preferably 0.05 - 100 mg, is administered to one adult in one to several portions a day. For parenteral administration such as intravenous administration and the like, 0.001 - 1000 mg, preferably 0.01 - 100 mg, is generally administered to one adult in one to several portions a day. For transdermal administration, an external preparation containing 0.001 - 10% of compound (I) or a pharmaceutically acceptable salt thereof is generally applied once to several times a day. However, these doses and administration frequencies vary depending on the aforementioned various conditions.

A combination of compound (IC) or a pharmaceutically acceptable salt thereof and one or more of other pharmaceutical components can also be used as the agent of the present invention for use in the treatment and/or prophylaxis of an anxiety disorder.

Examples of other pharmaceutical component to be used in the combination include other drugs having an antianxiety action, for example, tryptamine reuptake inhibitors such as buspirone, sertraline, paroxetine, nefazodone, fluoxetine and the like; GABA receptor agonists such as benzodiazepine and the like (for example, diazepam, tofisopam, alprazolam, flutoprazepam and the like); corticotropin releasing factor antagonists such as pivagabine and the like; MAO inhibitors such as amisulpride and the like; and the like.

When compound (IC) or a pharmaceutically acceptable salt thereof is used in combination with the above-mentioned other pharmaceutical component, compound (IC) or a pharmaceutically acceptable salt thereof and other pharmaceutical component can be administered as a single preparation or a combination of plural preparations to patients in need thereof, as long as these components can be formulated as preparations, and a combination of two or more of

preparations is preferred. Furthermore, when compound (IC) or a pharmaceutically acceptable salt thereof and other pharmaceutical component are used or administered as a combination of plural preparations, these preparations can be used or administered simultaneously or separately at an interval.

When compound (IC) or a pharmaceutically acceptable salt thereof and other pharmaceutical component are administered as a combination of plural preparations, for example, a first component (a) containing compound (IC) or a pharmaceutically acceptable salt thereof, and a second component (b) containing other pharmaceutical component(s) are separately formulated, and prepared into a kit. Using the kit, each component may be administered to the same subject in the same route or in different routes simultaneously or separately at an interval.

As the kit, for example, a kit comprising contents and two or more containers (e.g., vials, bags, etc.) whose material, shape, and so on are not particularly limited as long as the containers do not cause degeneration of the components which are the contents due to external temperature or light nor cause elution of chemical components from the containers during storage, and having a form which enables the administration of the above first and second components which are the contents through separate routes (e.g., tubes, etc.) or the same route is used. Specific examples thereof include tablet kits, injection kits, and the like.

The following more specifically describes the present invention by way of Examples.

Example 1

Tablets having the following formulations are prepared according to the conventional manner. Compound (IA) (40 g), lactose (286.8 g), and potato starch (60 g) are mixed, and then a 10% aqueous solution of hydroxypropylcellulose (120 g) is added thereto. The resulting mixture is kneaded according to the conventional manner, granulated, and dried to form granules for tableting. After adding thereto 1.2 g of magnesium stearate followed by mixing, the mixture is punched with a tableting machine having a punch measuring 8 mm in diameter (Model RT-15; Kikusui) to obtain tablets (containing 20 mg of an active ingredient per tablet).

[Table 3]

| Formulation | | |
|---|---|---|
| compound (IA)* | 20 | mg |
| lactose | 143.4 | mg |
| potato starch | 30 | mg |
| hydroxypropylcellulose | 6 | mg |
| magnesium stearate | 0.6 | mg |
| | 200 | mg |

** not encompassed by the present invention*

Example 2

Tablets having the following formulation are prepared in the same manner as in Example 1.

[Table 4]

| Formulation | | |
|---|---|---|
| compound (IB)* | 20 | mg |
| lactose | 143.4 | mg |
| potato starch | 30 | mg |
| hydroxypropylcellulose | 6 | mg |
| magnesium stearate | 0.6 | mg |
| | 200 | mg |

** not encompassed by the present invention*

Example 3

Tablets having the following formulation are prepared in the same manner as in Example 1.

[Table 5]

| Formulation | | |
|---|---|---|
| compound (IC) | 20 | mg |
| lactose | 143.4 | mg |
| potato starch | 30 | mg |
| hydroxypropylcellulose | 6 | mg |
| magnesium stearate | 0.6 | mg |
| | 200 | mg |

Example 4

Injections having the following formulation are prepared according to the conventional manner. Compound (IA)* (1 g) is added to distilled water for injection followed by mixing. After adjusting the pH of the mixture to 7 by adding ** not encompassed by the present invention* hydrochloric acid and a sodium hydroxide aqueous solution thereto, the total volume is adjusted to 1,000 mL with distilled water for injection. The resulting mixture is aseptically charged into glass vials in 2-mL portions to obtain injections (containing 2 mg of an active ingredient per vial).

[Table 6]

| Formulation | |
|---|---|
| compound (IA)* | 2 mg |
| hydrochloric acid | Appropriate amount |
| aqueous sodium hydroxide solution | Appropriate amount |
| distilled water for injection | Appropriate amount |
| | 2.00 mL |
| ** not encompassed by the present invention* | |

Example 5

In the same manner as in Example 4, an injection having the following composition is prepared.

[Table 7]

| Formulation | |
|---|---|
| compound (IB)* | 2 mg |
| hydrochloric acid | Appropriate amount |
| aqueous sodium hydroxide solution | Appropriate amount |
| distilled water for injection | Appropriate amount |
| | 2.00 mL |
| ** not encompassed by the present invention* | |

Example 6

In the same manner as in Example 4, an injection having the following composition is prepared.

[Table 8]

| Formulation | |
|---|---|
| compound (IC) | 2 mg |
| hydrochloric acid | Appropriate amount |
| aqueous sodium hydroxide solution | Appropriate amount |
| distilled water for injection | Appropriate amount |
| | 2.00 mL |

Reference Example 1

Compounds (IA) - (ID) were obtained according to the method described in WO2005/063743. Only compound (IC) is a compound for use in accordance with the present invention.

Reference Example 2

N-[4-(2-Furyl)-5-(tetrahydropyran-4-carbonyl)thiazol-2-yl)-6-vinylpyridine-3-carboxamide (compound (IE))

step 1 Methyl 6-chloronicotinate (1.51 g, 8.79 mmol) was dissolved in DMF (35 mL), vinyltributyltin (3.32 mL, 11.4 mmol), dichlorobis(tri-o-tolylphosphine)palladium (206 mg, 0.262 mmol) and lithium chloride (554 mg, 13.1 mmol) were added and the mixture was stirred at 100°C for 2 hr. The mixture was allowed to cool to room temperature, and an aqueous potassium fluoride solution was added thereto. The mixture was filtered through Celite and the residue was washed with ethyl acetate. To the obtained filtrate was added a saturated aqueous sodium hydrogen carbonate solution, and the mixture was extracted with ethyl acetate. The organic layer was washed with saturated brine, dried over anhydrous magnesium sulfate, and concentrated under reduced pressure. The obtained residue was purified by silica gel column chromatography (hexane:ethyl acetate=70:30) to give methyl 6-vinylnicotinate (1.22 g, 85%) as a colorless transparent oil.
$^1$H NMR ($CDCl_3$, $\delta$ppm) : 3.95 (s, 3H), 5.63 (dd, J = 1.1, 10.8 Hz, 1H), 6.35 (dd, J = 1.1, 17.4 Hz, 1H), 6.87 (dd, J = 10.8, 17.4 Hz, 1H), 7.40 (d, J = 8.2 Hz, 1H), 8.25 (dd, J = 2.1, 8.2 Hz, 1H), 9.15-9.18 (m,1H).

step 2 Methyl 6-vinylnicotinate (491 mg, 2.97 mmol) obtained above was dissolved in a 50% methanol aqueous solution (8 mL). Lithium hydroxide monohydrate (276 mg, 6.57 mmol) was added thereto and the mixture was stirred at room temperature for 1 hr. The mixture was cooled to 0°C, then 3 mol/L hydrochloric acid (3 mL) was added, and the precipitated solid was collected by filtration to give 6-vinylnicotinic acid (309 mg, 70%) as a white solid.
$^1$H NMR (DMSO-$d_6$, $\delta$ppm): 5.61 (dd, J = 1.5, 10.8 Hz, 1H), 6.37 (dd, J = 1.5, 17.4 Hz, 1H), 6.89 (dd, J = 10.8, 17.4 Hz, 1H), 7.62 (d, J = 8.2 Hz, 1H), 8.22 (dd, J = 2.2, 8.2 Hz, 1H), 9.01 (d, J = 2.2 Hz, 1H), 13.35 (brs, 1H).

step 3 2-Amino-4-(2-furyl)thiazol-5-yl=tetrahydropyran-4-yl=ketone (301 mg, 1.08 mmol) described in WO2005/063743 was dissolved in DMF (1.5 mL), EDC hydrochloride (412 mg, 2.15 mmol), DMAP (66 mg, 0.54 mmol) and 6-vinylnicotinic acid (306 mg, 1.65 mmol) were added thereto, and the mixture was stirred at 50°C for 5 hr. The mixture was allowed to cool to room temperature, water and a saturated aqueous sodium hydrogen carbonate solution were added thereto and the mixture was extracted with ethyl acetate. The organic layer was washed with saturated brine, dried over anhydrous magnesium sulfate, and concentrated under reduced pressure. The obtained residue was purified by silica gel column chromatography (hexane:ethyl acetate=50:50), and recrystallized from ethanol-water to give compound (IE) (1.22 g, 85%) as white crystals.
$^1$H NMR ($CDCl_3$, $\delta$ppm): 1.80-2.01 (m, 4H), 3.11-3.25 (m, 1H), 3.51 (ddd, J = 3.1, 11.4, 11.4 Hz, 2H), 4.02-4.11 (m, 2H), 5.71 (dd, J = 0.8, 10.7 Hz, 1H), 6.43 (dd, J = 0.8, 17.5 Hz, 1H), 6.57 (dd, J = 1.7, 3.8 Hz, 1H), 6.90 (dd, J = 10.7, 17.5 Hz, 1H), 7.51 (d, J = 8.2 Hz, 1H), 7.58 (dd, J = 0.5, 1.7 Hz, 1H), 7.84 (d, J = 3.8 Hz, 1H), 8.21 (dd, J = 2.4, 8.2 Hz, 1H), 9.13 (d, J = 2.4 Hz, 1H), 9.84 (brs, 1H). ESIMS m/z: $[M+H]^+$ 410.

Reference Example 3

N-[4-(2-Furyl)-5-(tetrahydropyran-4-carbonyl)thiazol-2-yl]-2-(pyridin-3-yl)acetamide (compound (IF))

2-Amino-4-(2-furyl)thiazol-5-yl=tetrahydropyran-4-yl=ketone (105 mg, 0.377 mmol) described in WO2005/063743 was dissolved in DMF (2.0 mL), EDC hydrochloride (421 mg, 2.20 mmol), HOBt monohydrate (340 mg, 2.21 mmol) and 3-pyridylacetic acid hydrochloride (370 mg, 2.14mmol) were added thereto, and the mixture was stirred at 80°C overnight. The mixture was allowed to cool to room temperature, and water and a saturated aqueous sodium hydrogen carbonate solution were added thereto. The precipitated solid was collected by filtration, and dried under reduced pressure. The obtained solid was purified by silica gel column chromatography (hexane:ethyl acetate=50:50), and recrystallized from ethanol-water to give compound (IF) (112 mg, 75%) as white crystals.
$^1$H NMR ($CDCl_3$, $\delta$ppm): 1.80-2.01 (m, 4H), 3.05-3.16 (m, 1H), 3.45 (ddd, J = 2.8, 11.4, 11.4 Hz, 2H), 3.81 (s, 2H), 3.97-4.06 (m, 2H), 6.54 (dd, J = 1.8, 3.6 Hz, 1H), 7.32 (dd, J = 7.8, 4.8 Hz, 1H), 7.52-7.54 (m, 1H), 7.62-7.68 (m, 2H), 8.55-8.64 (m, 2H), 9.21 (s, 1H). APCIMS m/z: $[M+H]^+$ 398.

Reference Example 4

N-[4-(2-Furyl)-5-(tetrahydropyran-4-carbonyl)thiazol-2-yl]-1H-pyrrole-2-carboxamide (compound (IG))

In the same manner as in Reference Example 3, compound (IG) (86.0 mg, 65%) was obtained as pale-brown

crystals from 2-amino-4-(2-furyl)thiazol-5-yl=tetrahydropyran-4-yl=ketone (100 mg, 0.360 mmol) described in WO2005/063743 and pyrrole-2-carboxylic acid (240 mg, 2.18 mmol).
$^1$H NMR ($CDCl_3$, δppm): 1.80-2.01 (m, 4H), 3.08-3.24 (m, 1H), 3.47 (ddd, J = 2.7, 11.5, 11.5 Hz, 2H), 4.00-4.09 (m, 2H), 6.34-6.36 (m, 1H), 6.56 (dd, J = 1.8, 3.6 Hz, 1H), 6.86-6.88 (m, 1H), 7.06-7.10 (m, 1H), 7.55-7.57 (m, 1H), 7.71 (dd, J = 0.7, 3.7 Hz, 1H), 9.49 (brs, 1H), 9.65 (brs, 1H). APCIMS m/z: $[M+H]^+$ 372.

Reference Example 5

N-[4-(2-Furyl)-5-(tetrahydropyran-4-carbonyl)thiazol-2-yl]-1H-indole-4-carboxamide (compound (IH))

In the same manner as in Reference Example 3, compound (IH) (97.6 mg, 63%) was obtained as milky white crystals from 2-amino-4-(2-furyl)thiazol-5-yl=tetrahydropyran-4-yl=ketone (102 mg, 0.367 mmol) described in WO2005/063743 and indole-4-carboxylic acid (331 mg, 2.05 mmol).
$^1$H NMR ($CDCl_3$, δppm): 1.80-2.01 (m, 4H), 3.17-3.28 (m, 1H), 3.50 (ddd, J = 3.0, 11.2, 11.2 Hz, 2H), 4.02-4.11 (m, 2H), 6.58 (dd, J = 1.7, 3.5 Hz, 1H), 7.23-7.36 (m, 2H), 7.43-7.48 (m, 1H), 7.58-7.60 (m, 1H), 7.67 (dd, J = 4.2, 7.7 Hz, 2H), 7.76 (dd, J = 0.7, 3.5 Hz, 1H), 8.46 (brs, 1H), 9.70 (brs, 1H). APCIMS m/z: $[M+H]^+$ 422.

Reference Example 6

N-[4-(2-Furyl)-5-(tetrahydropyran-4-carbonyl)thiazol-2-yl]-2-(morpholin-4-ylmethyl)pyridine-4-carboxamide (compound (II))

step 1 2-Amino-4-(2-furyl)thiazol-5-yl=tetrahydropyran-4-yl=ketone (2.00 g, 7.19 mmol)described in WO2005/063743 was dissolved in DMF (35 mL), EDC hydrochloride (5.50 g, 28.6 mmol), HOBt monohydrate (4.40 g, 28.8 mmol) and 2-(chloromethyl)isonicotinic acid (4.93 g, 28.7 mmol) obtained by the method described in WO03/043636 were added thereto, and the mixture was stirred at 80°C overnight. The mixture was allowed to cool to room temperature, and water and a saturated aqueous sodium hydrogen carbonate solution were added thereto. The precipitated solid was collected by filtration, and dried under reduced pressure. The obtained solid was purified by silica gel column chromatography (hexane:ethyl acetate=50:50) to give 2-(chloromethyl)-N-[4-(2-furyl)-5-(tetrahydropyran-4-carbonyl)thiazol-2-yl]pyridine-4-carboxamide (700 mg, 23%) as a pale-brown solid.
$^1$H NMR ($CDCl_3$, δppm): 1.84-1.97 (m, 4H), 3.12-3.23 (m, 1H), 3.46-3.57 (m, 2H), 4.02-4.11 (m, 2H), 4.75 (s, 2H), 6.52 (dd, J = 3.6, 1.7 Hz, 1H), 7.50 (dd, J = 1.7, 0.7 Hz, 1H), 7.70 (dd, J = 5.1, 1.7 Hz, 1H), 7.79 (dd, J = 3.6, 0.7 Hz, 1H), 7.92-7.95 (m, 1H), 8.79 (dd, J = 5.1, 0.7 Hz, 1H).
step 2 2-(Chloromethyl)-N-[4-(2-furyl)-5-(tetrahydropyran-4-carbonyl)thiazol-2-yl]pyridine-4-carboxamide (70.0 mg, 0.162 mmol) obtained in step 1 was dissolved in acetonitrile (2.0 mL), then morpholine (70.0 μL, 2.15 mmol) was added thereto, and the mixture was stirred with heating under reflux for 1 hr. The mixture was allowed to cool to room temperature, water and a saturated aqueous sodium hydrogen carbonate solution were added thereto. The mixture was extracted with ethyl acetate, and the organic layer was washed with saturated brine, dried over anhydrous magnesium sulfate, and concentrated under reduced pressure. The obtained residue was purified by silica gel column chromatography (chloroform:methanol=95:5), and reslurried with hexane-ethyl acetate to give compound (II) (54.6 mg, 71%) as a pale-brown solid.
$^1$H NMR ($CDCl_3$, δppm): 1.80-2.01 (m, 4H), 2.51-2.59 (m, 4H), 3.10-3.24 (m, 1H), 3.51 (ddd, J = 3.0, 11.3, 11.3 Hz, 2H), 3.75-3.82 (m, 6H), 4.01-4.13 (m, 2H), 6.59 (dd, J = 1.8, 3.6 Hz, 1H), 7.60 (dd, J = 0.7, 1.8 Hz, 1H), 7.69 (dd, J = 1.8, 5.1 Hz, 1H), 7.84 (dd, J = 0.7, 3.6 Hz, 1H), 7.93-7.95 (m, 1H), 8.82 (dd, J = 0.7, 5.1 Hz, 1H). ESIMS m/z: $[M+H]^+$ 483.

Reference Example 7

N-[4-(2-Furyl)-5-(tetrahydropyran-4-carbonyl)thiazol-2-yl]-2-methoxymethylpyridine-4-carboxamide (compound (IJ))

Under ice-cooling, 60% sodium hydride (10.0 mg, 0.250 mmol) was dissolved in DMF (1.0 mL), methanol (110 μL, 2.72 mmol) was slowly added dropwise thereto, and the mixture was stirred at 0°C for 10 min. Then, 2-(chloromethyl)-N-[4-(2-furyl)-5-(tetrahydropyran-4-carbonyl)thiazol-2-yl]pyridine-4-carboxamide (81.0 mg, 0.189 mmol) obtained in step 1 of Reference Example 6, which was dissolved in DMF (1.0 mL), was slowly added dropwise thereto, and the mixture was stirred at room temperature for 5 hr. To the mixture were added water and a saturated aqueous sodium hydrogen carbonate solution, and the mixture was extracted with ethyl acetate. The organic layer was washed with saturated brine, dried over anhydrous magnesium sulfate, and concentrated under reduced pressure. The obtained residue was purified by silica gel column chromatography (hexane:ethyl acetate=50:50), and recrystallized from ethanol-water to give compound (IJ) (45.0 mg, 56%) as white crystals.

$^1$H NMR ($CDCl_3$, δppm): 1.80-2.01 (m, 4H), 3.14-3.23 (m, 1H), 3.52 (ddd, J = 3.0, 11.2, 11.2 Hz, 2H), 3.53 (s, 3H), 4.02-4.18 (m, 2H), 4.5 (s, 2H), 6. 52 (dd, J = 1.8, 3.6 Hz, 1H), 7.50 (d, J = 1.1 Hz, 1H), 7.71 (dd, J = 1.3, 5.1 Hz, 1H), 7.79 (d, J = 3.6 Hz, 1H), 7.85 (s, 1H), 8.77 (d, J = 5.1 Hz, 1H), 10.41 (brs, 1H). APCIMS m/z: $[M+H]^+$ 428.

Reference Example 8

2-Ethoxymethyl-N-[4-(2-furyl)-5-(tetrahydropyran-4-carbonyl)thiazol-2-yl]pyridine-4-carboxamide (compound (IK))

In the same manner as in Reference Example 7, compound (IK) (47.0 mg, 57%) was obtained as white crystals from 2-(chloromethyl)-N-[4-(2-furyl)-5-(tetrahydropyran-4-carbonyl)thiazol-2-yl]pyridine-4-carboxamide (80.0 mg, 0.185 mmol) and ethanol (200 μL, 3.54 mmol).
$^1$H NMR ($CDCl_3$, δppm): 1.36 (t, J = 7.1 Hz, 3H), 1.80-2.01 (m, 4H), 3.11-3.28 (m, 1H), 3.51 (ddd, J = 3.2, 11.4, 11.4 Hz, 2H), 3.72 (q, J = 7.1 Hz, 2H), 4.00-4.12 (m, 2H), 4.73 (s, 2H), 6.58 (dd, J = 1.7, 3.6 Hz, 1H), 7.58 (dd, J = 0.7, 1.7 Hz, 1H), 7.72 (dd, J = 1.7, 5.0Hz, 1H), 7.84 (dd, J = 0.7, 3.6 Hz, 1H), 7.92 (dd, J = 0.7, 1.7Hz, 1H), 8.80 (d, J = 5.0 Hz, 1H), 9.95 (brs, 1H). APCIMS m/z: $[M+H]^+$ 442.

Reference Example 9

N-[4-(2-Furyl)-5-(tetrahydropyran-4-carbonyl)thiazol-2-yl]-2-isopropoxymethylpyridine-4-carboxamide (compound (IL))

In the same manner as in Reference Example 7, compound (IL) (30.2 mg, 36%) was obtained as white crystals from 2-(chloromethyl)-N-[4-(2-furyl)-5-(tetrahydropyran-4-carbonyl)thiazol-2-yl]pyridine-4-carboxamide (80.1 mg, 0.185 mmol) and 2-propanol (350 μL, 4.60 mmol).
$^1$H NMR ($CDCl_3$, δppm): 1.31 (d, J = 6.0 Hz, 6H), 1.80-2.01 (m, 4H), 3.15-3.22 (m, 1H), 3.51 (ddd, J = 2.8, 11.4, 11.4 Hz, 2H), 3.78-3.86 (qq, J = 6.0, 6.0 Hz, 1H), 4.01-4.11 (m, 2H), 4.73 (s, 2H), 6.58 (dd, J = 1.8, 3.6 Hz, 1H), 7.59 (dd, J = 0.6, 1.8 Hz, 1H), 7.71 (dd, J = 1.5, 5.1 Hz, 1H), 7.85 (dd, J = 0.4, 3.5 Hz, 1H), 7.93 (d, J = 0.6 Hz, 1H), 8.79 (dd, J = 0.4, 5.1 Hz, 1H), 9.91 (brs, 1H). APCIMS m/z: $[M+H]^+$ 456.

Reference Example 10

N-[4-(2-Furyl)-5-(tetrahydropyran-4-carbonyl)thiazol-2-yl]furo[2,3-b]pyridine-5-carboxamide (compound (IM))

2-Amino-4-(2-furyl)thiazol-5-yl=tetrahydropyran-4-yl=ketone (125 mg, 0.450 mmol) described in WO2005/063743 was dissolved in DMF (2.2 mL), EDC hydrochloride (173 mg, 0.900 mmol), HOBt monohydrate (138 mg, 0.900 mmol) and furo[2,3-b]pyridine-5-carboxylic acid (147 mg, 0.900 mmol) obtained in the method described in Tetrahedron Letters, vol. 35, p.9355 (1994) were added thereto, and the mixture was stirred at 50°C for 2 hr, then at 70°C for 1 hr. To the mixture were added EDC hydrochloride (173 mg, 0.900 mmol), HOBt monohydrate (138 mg, 0.900 mmol) and furo[2,3-b]pyridine-5-carboxylic acid (147 mg, 0.900 mmol), and the mixture was stirred at 70°C for 1.5 hr. The mixture was added to water- a saturated aqueous sodium hydrogen carbonate solution (1:1) and the precipitated solid was collected by filtration and dried. The obtained solid was purified by silica gel column chromatography (hexane:ethyl acetate=50:50), and recrystallized from ethanol-water to give compound (IM) (81.2 mg, 43%).
$^1$H NMR (DMSO-$d_6$, δppm): 1.56-1.77 (m, 4H), 3.16-3.26 (m, 1H), 3.37-3.47 (m, 2H), 3.87-3.92 (m, 2H), 6.71 (dd, J = 1.9, 3.5 Hz, 1H), 7.21 (d, J = 2.4 Hz, 1H), 7.45 (dd, J = 0.9, 3.5 Hz, 1H), 7.91 (dd, J = 0.9, 1.9 Hz, 1H), 8.27 (d, J = 2.4 Hz, 1H), 8.86 (d, J = 2.4 Hz, 1H), 9.04 (d, J = 2.4 Hz, 1H). ESIMS m/z: $[M+H]^+$ 424.

Reference Example 11

N-[4-(2-Furyl)-5-(tetrahydropyran-4-carbonyl)thiazol-2-yl]-2-(pyridin-2-yl)acetamide (compound (IN))

In the same manner as in step 3 of Reference Example 2, compound (IN) (125 mg, 58%) was obtained as white crystals from 2-amino-4-(2-furyl)thiazol-5-yl=tetrahydropyran-4-yl=ketone (154 mg, 0.553 mmol) described in WO2005/063743 and 2-pyridylacetic acid hydrochloride (196 mg, 1.13 mmol).
$^1$H NMR ($CDCl_3$, 5ppm): 1.78-1.95 (m, 4H), 3.01-3.21 (m, 1H), 3.47 (ddd, J = 2.6, 11.4, 11.4 Hz, 2H), 3.98-4.09 (m, 2H), 4.03 (s, 2H), 6.57 (dd, J = 1.8, 3.6 Hz, 1H), 7.25-7.34 (m, 2H), 7.59 (dd, J = 0.7, 1.8 Hz, 1H), 7.70 (dd, J = 0.7, 3.5 Hz, 1H), 7.74 (ddd, J = 1.8, 7.7, 7.7 Hz, 1H), 8.69-8.73 (m, 1H), 12.09 (brs, 1H). APCIMS m/z: $[M+H]^+$ 398.

Reference Example 12

N-[4-(2-Furyl)-5-(tetrahydropyran-4-carbonyl)thiazol-2-yl]-6-methoxypyridine-3-carboxamide (compound (IO))

In the same manner as in step 3 of Reference Example 2, compound (IO) (121 mg, 54%) was obtained as white crystals from 2-amino-4-(2-furyl)thiazol-5-yl=tetrahydropyran-4-yl=ketone (150 mg, 0.539 mmol) described in WO2005/063743 and 6-methoxynicotinic acid (101 mg, 0.659 mmol).
$^1$H NMR ($CDCl_3$, δppm): 1.80-2.01 (m, 4H), 3.10-3.25 (m, 1H), 3.51 (ddd, J = 2.9, 11.4, 11.4 Hz, 2H), 4.02-4.11 (m, 2H), 4.04 (s, 3H), 6.55 (dd, J = 1.7, 3.5 Hz, 1H), 6.87 (d, J = 8.8 Hz, 1H), 7.53-7.57 (m, 1H), 7.83 (dd, J = 0.6, 3.5 Hz, 1H), 8.10 (dd, J = 2.6, 8.8 Hz, 1H), 8.77 (dd, J = 0.6, 2.6 Hz, 1H), 9.93 (brs, 1H). APCIMS m/z: $[M+H]^+$ 414.

Reference Example 13

N-[4-(2-Furyl)-5-(tetrahydropyran-4-carbonyl)thiazol-2-yl]quinoline-3-carboxamide (compound (IP))

In the same manner as in step 3 of Reference Example 2, compound (IP) (178 mg, 76%) was obtained as pale-yellow crystals from 2-amino-4-(2-furyl)thiazol-5-yl=tetrahydropyran-4-yl=ketone (151 mg, 0.543 mmol) described in WO2005/063743 and quinoline-3-carboxylic acid (142 mg, 0.820 mmol).
$^1$H NMR ($CDCl_3$, δppm): 1.80-2.01 (m, 4H), 3.15-3.25 (m, 1H), 3.52 (ddd, J = 2.9, 11.4, 11.4 Hz, 2H), 4.06-4.10 (m, 2H), 6.47 (dd, J = 1.7, 3.5 Hz, 1H), 7.47 (dd, J = 0.7, 1.6 Hz, 1H), 7.66-7.74 (m, 2H), 7.87-7.95 (m, 2H), 8.20 (dd, J = 0.9, 8.4 Hz, 1H), 8.71 (d, J = 1.8 Hz, 1H), 9.43 (d, J = 2.4 Hz, 1H), 10.55 (s, 1H). APCIMS m/z: $[M+H]^+$ 434.

Reference Example 14

N-[4-(2-Furyl)-5-(tetrahydropyran-4-carbonyl)thiazol-2-yl]-5,6-dimethylpyridine-3-carboxamide (compound (IQ))

step 1 5,6-Dimethylpyridine-3-carbonitrile (502 mg, 3.79 mmol) obtained by the method described in J. Heterocyclic Chem., vol. 24, p. 351 (1987) was suspended in 70% aqueous ethanol (4.5 mL), sodium hydroxide (444 mg, 11.1 mmol) was added thereto, and the mixture was stirred with heating under reflux for 3 hr. The mixture was ice-cooled to 0°C, and 6 mol/L hydrochloric acid (1.9 mL) was added thereto. The mixture was concentrated under reduced pressure and the obtained residue was suspended in chloroform-methanol. The inorganic salt was removed by filtration, and the obtained filtrate was concentrated under reduced pressure to give 5,6-dimethylpyridine-3-carboxylic acid (569 mg, 99%) as a pale-pink solid.
$^1$H NMR (DMSO-$d_6$, δppm): 2.23 (s, 3H), 2.39 (s, 3H), 7.83 (d, J = 1.7 Hz, 1H), 8.64 (d, J = 1.7 Hz, 1H).
step 2 In the same manner as in step 3 of Reference Example 2, compound (IQ) (112 mg, 49%) was obtained as white crystals from 2-amino-4-(2-furyl)thiazol-5-yl=tetrahydropyran-4-yl=ketone (151 mg, 0.550 mmol) described in WO2005/063743 and 5,6-dimethylpyridine-3-carboxylic acid (166 mg, 1.10 mmol) obtained above.
$^1$H NMR ($CDCl_3$, δppm): 1.80-2.01 (m, 4H), 2.34 (s, 3H), 2.59 (s, 3H), 3.12-3.23 (m, 1H), 3.51 (ddd, J = 2.9, 11.3, 11.3 Hz, 2H), 4.04-4.09 (m, 2H), 6.49 (dd, J = 2.0, 3.6 Hz, 1H), 7.47 (d, J = 1.7 Hz, 1H), 7.79 (dd, J = 0.5, 3.5 Hz, 1H), 7.89 (d, J = 1.7 Hz, 1H), 8.86 (d, J = 2.0 Hz, 1H). ESIMS m/z: $[M+H]^+$ 412.

Reference Example 15

5-Ethyl-N-[4-(2-furyl)-5-(tetrahydropyran-4-carbonyl)thiazol-2-yl]pyridine-3-carboxamide (compound (IR))

In the same manner as in step 3 of Reference Example 2, compound (IR) (145 mg, 65%) was obtained as white crystals from 2-amino-4-(2-furyl)thiazol-5-yl=tetrahydropyran-4-yl=ketone (151 mg, 0.543 mmol) described in WO2005/063743 and 5-ethylnicotinic acid (128 mg, 0.814 mmol).
$^1$H NMR ($CDCl_3$, δppm): 1.32 (t, J = 7.6 Hz, 3H), 1.83-2.01 (m, 4H), 2.77 (q, J = 7.6 Hz, 2H), 3.11-3.26 (m, 1H), 3.51 (ddd, J = 2.9, 11.4, 11.4 Hz, 2H), 4.01-4.11 (m, 2H), 6.54 (dd, J = 1.8, 3.6 Hz, 1H), 7.51-7.53 (m, 1H), 7.80 (dd, J = 0.7, 3.6 Hz, 1H), 8.03-8.06 (m, 1H), 8.70 (d, J = 2.0 Hz, 1H), 8.99 (d, J = 2.0 Hz, 1H), 10.24 (brs, 1H). ESIMS m/z: $[M+H]^+$ 412.

Reference Example 16

N-[4-(2-Furyl)-5-(tetrahydropyran-4-carbonyl)thiazol-2-yl]-7,8-dihydro-5H-pyrano[4,3-b]pyridine-3-carboxamide (compound (IS))

step 1 Sodium hydride (2.06 g, 51.5 mmol) was suspended in diethyl ether (40 mL), and methanol (2.1 mL,

51.8 mmol) was added slowly at -5°C thereto. To the mixture was added ethanol (6 mL), and the mixture was stirred at room temperature for 5 min, and cooled to 0°C. A mixture of tetrahydro-4H-pyran-4-one (4.61 mL, 49.9 mmol) and ethyl formate (4.11 mL, 51.1 mmol) was slowly added thereto. The mixture was stirred at room temperature for 2 hr, and the resultant product was extracted with water (30 mL) (aqueous solution A).

Then, an aqueous piperidine - acetic acid solution prepared by dissolving acetic acid (1.5 mL) in water (3.5 mL) and adding piperidine (2.6 mL) thereto, and 2-cyanoacetamide (4.62 g, 54.9 mmol) were added to the above-mentioned aqueous solution A, and the mixture was stirred with heating under reflux for 4 hr. To the mixture was added acetic acid (3.6 mL) and, after cooling 0°C, the precipitated solid was collected by filtration to give 2-oxo-1,5,7,8-tetrahydro-2H-pyrano[4,3-b]pyridine-3-carbonitrile (1.72 g, 20%) as a white solid.

$^1$H NMR ($CDCl_3$, δppm): 2.89 (t, J = 5.6 Hz, 2H), 3.99 (t, J = 5.6 Hz, 2H), 4.54 (s, 2H), 7.59 (s, 1H). APCIMS m/z: $[M-H]^-$ 175.

step 2 2-Oxo-1,5,7,8-tetrahydro-2H-pyrano[4,3-b]pyridine-3-carbonitrile (2.50 g, 14.4 mmol) obtained in step 1 was dissolved in phosphoryl chloride (20 mL), and the mixture was stirred with heating under reflux for 4 hr. The mixture was allowed to cool to room temperature, and slowly added to a saturated aqueous sodium hydrogen carbonate solution at 0°C, then the mixture was extracted with chloroform. The organic layer was washed with saturated brine, dried over anhydrous magnesium sulfate, and concentrated under reduced pressure. The obtained residue was purified by silica gel column chromatography (hexane:ethyl acetate=50:50) to give 2-chloro-7,8-dihydro-5H-pyrano[4,3-b]pyridine-3-carbonitrile (1.85 g, 66%) as a white solid.

$^1$H NMR ($CDCl_3$, δppm): 3.07 (t, J = 5.8 Hz, 2H), 4.07 (t, J = 5.8 Hz, 2H), 4.75-4.76 (m, 2H), 7.63 (s, 1H).

step 3 2-Chloro-7,8-dihydro-5H-pyrano[4,3-b]pyridine-3-carbonitrile (1.77 g, 9.09 mmol) obtained in step 2 was dissolved in ethanol (30 mL), acetic acid (9 mL) and zinc (2.60 g) were added thereto, and the mixture was stirred with heating under reflux for 4 hr. The mixture was allowed to cool to room temperature, then filtered through Celite, and the filtrate was concentrated under reduced pressure. To the obtained residue was added a saturated aqueous sodium hydrogen carbonate solution and the mixture was extracted with chloroform. The organic layer was washed with saturated brine, dried over anhydrous magnesium sulfate, and concentrated under reduced pressure. The obtained residue was purified by silica gel column chromatography (hexane:ethyl acetate=50:50) to give 7,8-dihydro-5H-pyrano[4,3-b]pyridine-3-carbonitrile (1.06 g, 73%) as a white solid.

$^1$H NMR ($CDCl_3$, δppm): 3.10 (t, J = 5.8 Hz, 2H), 4.10 (t, J = 5.8 Hz, 2H), 4.79 (s, 2H), 7.59 (d, J = 1.7 Hz, 1H), 8.71 (d, J = 1.7 Hz, 1H). APCIMS m/z: $[M+H]^+$ 161.

step 4 In the same manner as in step 1 of Reference Example 14, 7,8-dihydro-5H-pyrano[4,3-b]pyridine-3-carboxylic acid (318 mg, 47%) was obtained as a white solid from 7,8-dihydro-5H-pyrano[4,3-b]pyridine-3-carbonitrile (609 mg, 3.80 mmol) obtained above.

$^1$H NMR (DMSO-$d_6$, δppm): 2.86 (t, J = 5.8 Hz, 2H), 3.95 (t, J = 5.8 Hz, 2H), 4.70 (s, 2H), 7.80 (d, J = 1.7 Hz, 1H), 8.76(d, J = 1.7 Hz, 1H). ESIMS m/z: $[M-H]^-$ 178.

step 5 In the same manner as in step 3 of Reference Example 2, compound (IS) (178 mg, 74%) was obtained as white crystals from 2-amino-4-(2-furyl)thiazol-5-yl=tetrahydropyran-4-yl=ketone (152 mg, 0.546 mmol) described in WO2005/063743 and 7,8-dihydro-5H-pyrano[4,3-b]pyridine-3-carboxylic acid (432 mg, 2.00 mmol) obtained above.

$^1$H NMR ($CDCl_3$, 5ppm): 1.80-2.01 (m, 4H), 3.10 (t, J = 5.6 Hz, 2H), 3.13-3.24 (m, 1H), 3.51 (ddd, J = 2.8, 11.4, 11.4 Hz, 2H), 4.03-4.14 (m, 4H), 4.79 (s, 2H), 6.50 (dd, J = 1.7, 3.6 Hz, 1H), 7.46 (dd, J = 0.6, 1.7 Hz, 1H), 7.78 (dd, J = 0.6, 3.6 Hz, 1H), 7.82 (d, J = 2.2 Hz, 1H), 8.94 (d, J = 2.2 Hz, 1H), 10.58 (s, 1H). ESIMS m/z: $[M+H]^+$ 440.

Reference Example 17

N-[4-(2-Furyl)-5-(tetrahydropyran-4-carbonyl)thiazol-2-yl]-6,7-dihydro-5H-cyclopenta[b]pyridine-3-carboxamide (compound (IT))

step 1 6,7-Dihydro-5H-cyclopenta[b]pyridine-3-carbonitrile (901 mg, 6.25 mmol) obtained by the method described in J. Heterocyclic Chem., vol. 24, p. 351 (1987) was suspended in 6 mol/L hydrochloric acid (9 mL), and the mixture was stirred with heating under reflux for 5 hr. The mixture was ice-cooled to 0°C, and the precipitated solid was collected by filtration to give 6,7-dihydro-5H-cyclopenta[b]pyridine-3-carboxylic acid hydrochloride (543 mg, 44%) as a pale-brown solid.

$^1$H NMR (DMSO-$d_6$, δppm): 2.16 (tt, J = 7.4, 7.8 Hz, 2H), 3.02 (t, J = 7.4 Hz, 2H), 3.10 (t, J = 7.8 Hz, 2H), 8.34 (s, 1H), 8.92 (s, 1H).

step 2 In the same manner as in step 3 of Reference Example 2, compound (IT) (134 mg, 58%) was obtained as white crystals from 2-amino-4-(2-furyl)thiazol-5-yl=tetrahydropyran-4-yl=ketone (152 mg, 0.546 mmol) described in WO2005/063743 and 6,7-dihydro-5H-cyclopenta[b]pyridine-3-carboxylic acid hydrochloride (165 mg, 0.827 mmol) obtained above.

$^1$H NMR ($CDCl_3$, δppm): 1.78-2.01 (m, 4H), 2.16-2.28 (m, 2H), 3.01 (t, J = 7.6 Hz, 2H), 3.10 (t, J = 7.7 Hz, 2H), 3.11-3.25 (m, 1H), 3.51 (ddd, J = 3.0, 11.4, 11.4 Hz, 2H), 4.00-4.10 (m, 2H), 6.52 (dd, J = 1.8, 3.6 Hz, 1H), 7.51 (dd, J = 0.7, 1. 7

Hz, 1H), 7.80 (dd, J = 0.7, 3.6 Hz, 1H), 7.95-8.00 (m, 1H), 8.87-8.91 (m, 1H), 10.20 (brs, 1H). ESIMS m/z: $[M+H]^+$ 424.

Reference Example 18

N-[4-(2-Furyl)-5-(tetrahydropyran-4-carbonyl)thiazol-2-yl]-1H-indole-2-carboxamide (compound (IU))

In the same manner as in Reference Example 3, compound (IU) (97.5 mg, 63%) was obtained as pale-brown crystals from 2-amino-4-(2-furyl)thiazol-5-yl=tetrahydropyran-4-yl=ketone (102 mg, 0.366 mmol) described in WO2005/063743 and indole-2-carboxylic acid (350 mg, 2.17 mmol).
$^1$H NMR ($CDCl_3$, δppm): 1.80-2.01 (m, 4H), 3.10-3.24 (m, 1H), 3.50 (ddd, J = 2.7, 11.5, 11.5 Hz, 2H), 4.01-4.11 (m, 2H), 6.59 (dd, J = 1.7, 3.5 Hz, 1H), 7.14 (dd, J = 0.9, 2.2 Hz, 1H), 7.19-7.25 (m, 1H), 7.36-7.43 (m, 1H), 7.46-7.52 (m, 1H), 7.60 (dd, J = 0.7, 1.7 Hz, 1H), 7.72-7.77 (m, 1H), 7.83 (dd, J = 0.7, 3.5 Hz, 1H), 9.21 (brs, 1H), 9.66 (brs, 1H). APCIMS *m/z:* $[M+H]^+$ 422.

Reference Example 19

6-Ethyl-N-[4-(2-furyl)-5-(tetrahydropyran-4-carbonyl)thiazol-2-yl]pyridine-3-carboxamide (compound (IV))

Compound (IE) (90.0 mg, 0.220 mmol) obtained in Reference Example 2 was dissolved in ethanol (10 mL) under an argon atmosphere, 10% palladium carbon (10%-Pd/C; containing water) (88.9 mg) was added thereto, and mixture was stirred at room temperature overnight under a hydrogen atmosphere. The mixture was filtered through Celite, and the filtrate was concentrated under reduced pressure. The obtained residue was purified by preparative thin layer chromatography (hexane:ethyl acetate=30:70), and recrystallized from ethanol-water to give compound (IV) (70.0 mg, 77%) as white crystals.
$^1$H NMR ($CDCl_3$, δppm): 1.36 (t, J = 7.6 Hz, 3H), 1.80-2.01 (m, 4H), 2.94 (q, J = 7.6 Hz, 2H), 3.11-3.27 (m, 1H), 3.51 (ddd, J = 3.0, 11.3, 11.3 Hz, 2H), 3.99-4.13 (m, 2H), 6.54 (dd, J = 1.7, 3.5 Hz, 1H), 7.35 (d, J = 8.1 Hz, 1H), 7.52 (dd, J = 0.7, 1.7 Hz, 1H), 7.81 (dd, J = 0.7, 3.6 Hz, 1H), 8.15 (dd, J = 2.2, 8.2 Hz, 1H), 9.08 (d, J = 2.2 Hz, 1H), 10.13 (brs, 1H). ESIMS m/z: $[M+H]^+$ 412.

Reference Example 20

N-[4-(2-Furyl)-5-(tetrahydropyran-4-carbonyl)thiazol-2-yl]-6-propylpyridine-3-carboxamide (compound (IW))

step 1 In the same manner as in step 1 of Reference Example 2, methyl 6-(1-propenyl)nicotinate (327 mg, 37%) was obtained as a colorless transparent oil from methyl 6-chloronicotinate (862 mg, 6.48 mmol) and allyltributyltin (2.20 mL, 7.09 mmol).
$^1$H NMR ($CDCl_3$, δppm): 1.97 (dd, J = 1.7, 6.8 Hz, 3H), 3.95 (s, 3H), 6.55 (dq, J = 1.7, 15.7 Hz, 1H), 6.92 (dq, J = 6.8, 15.7 Hz, 1H), 7.25-7.30 (m, 1H), 8.19 (dd, J = 2.2, 8.2 Hz, 1H), 9.11 (dd, J = 0.5, 2.2 Hz, 1H).
step 2 In the same manner as in step 2 of Reference Example 2, 6-(1-propenyl)nicotinic acid (251 mg, 84%) was obtained as milk-white crystals from methyl 6-(1-propenyl)nicotinate (326 mg, 1.84 mmol) obtained above.
$^1$H NMR (DMSO-$d_6$, δppm): 1.91 (dd, J = 1.8, 6.8 Hz, 3H), 6.58 (dq, J = 1.8, 15.5 Hz, 1H), 6.91 (dq, J = 6.8, 15.5 Hz, 1H), 7.48 (dd, J = 0.5, 8.3 Hz, 1H), 8.15 (dd, J = 2.2, 8.3 Hz, 1H), 8.95 (dd, J = 0.5, 2.2 Hz, 1H), 13.24 (brs, 1H). ESIMS m/z: $[M+H]^+$ 164.
step 3 In the same manner as in step 3 of Reference Example 2, N-[4-(2-furyl)-5-(tetrahydropyran-4-carbonyl)thiazol-2-yl]-6-(1-propenyl)pyridine-3-carboxamide (125 mg, 33%) was obtained as white crystals from 2-amino-4-(2-furyl)thiazol-5-yl=tetrahydropyran-4-yl=ketone (257 mg, 0.908 mmol) described in WO2005/063743 and 6-(1-propenyl)nicotinic acid (251 mg, 1.26 mmol) obtained above.
$^1$H NMR ($CDCl_3$, δppm): 1.82-1.96 (m, 4H), 2.01 (dd, J = 1.4, 6.8 Hz, 3H), 3.12-3.23 (m, 1H), 3.52 (ddd, J = 3.0, 11.2, 11.2 Hz, 2H), 4.02-4.11 (m, 2H), 6.54-6.62 (m, 2H), 7.00 (dd, J = 6.8, 15.5 Hz, 1H), 7.37 (d, J = 8.4 Hz, 1H), 7.55 (dd, J = 0.8, 1.6 Hz, 1H), 7.82 (d, J = 3.6 Hz, 1H), 8.15 (dd, J = 2.4, 8.3 Hz, 1H), 9.08 (d, J = 2.4 Hz, 1H), 10.00 (brs, 1H). ESIMS m/z: $[M+H]^+$ 424.
step 4 In the same manner as in Reference Example 19, the title compound (IW) (96.0 mg, 76%) was obtained as white crystals from N-[4-(2-furyl)-5-(tetrahydropyran-4-carbonyl)thiazol-2-yl]-6-(1-propenyl)pyridine-3-carboxamide (125 mg, 0.296 mmol) obtained above.
$^1$H NMR ($CDCl_3$, δppm): 1.00 (t, J = 7.3 Hz, 3H), 1.75-1.97 (m, 6H), 2.88 (t, J = 7.6 Hz, 2H), 3.13-3.24 (m, 1H), 3.51 (ddd, J = 3.1, 11.4, 11.4 Hz, 2H), 4.02-4.11 (m, 2H), 6.55 (dd, J = 1.8, 3.6 Hz, 1H), 7.33 (d, J = 8.2 Hz, 1H), 7.53-7.55 (m, 1H), 7.81 (d, J = 3.6 Hz, 1H), 8.15 (dd, J = 2.5, 8.2 Hz, 1H), 9.09 (d, J = 2.1 Hz, 1H), 10.14 (s, 1H). ESIMS m/z: $[M+H]^+$ 426.

Reference Example 21

N-[4-(2-Furyl)-5-(tetrahydropyran-4-carbonyl)thiazol-2-yl]-7,8-dihydro-5H-thiopyrano[4,3-b]pyridine-3-carboxamide (compound (IX))

step 1 In the same manner as in step 1 of Reference Example 16, 2-oxo-1,5,7,8-tetrahydro-5H-thiopyrano[4,3-b]pyridine-3-carbonitrile (3.06 g, 37%) was obtained as a pale-yellow solid from tetrahydro-4H-thiopyran-4-one (5.00 g, 43.0 mmol).
$^1$H NMR ($CDCl_3$, δppm): 2.93 (t, J = 6.0 Hz, 2H), 3.11 (t, J = 6.0 Hz, 2H), 3.58 (s, 2H), 7.67 (s, 1H), 13.4 (brs, 1H).
step 2 In the same manner as in step 2 of Reference Example 16, 2-chloro-7,8-dihydro-5H-thiopyrano[4,3-b]pyridine-3-carbonitrile (1.75 g, 58%) was obtained from 2-oxo-1,5,7,8-tetrahydro-5H-thiopyrano[4,3-b]pyridine-3-carbonitrile (2.78 g, 14.4 mmol) obtained above.
$^1$H NMR ($CDCl_3$, δppm): 3.01 (t, J = 6.1 Hz, 2H), 3.27 (t, J = 6.1 Hz, 2H), 3.78 (s, 2H), 7.71 (s, 1H).
step 3 In the same manner as in step 3 of Reference Example 16, 7,8-dihydro-5H-thiopyrano[4,3-b]pyridine-3-carbonitrile (804 mg, 55%) was obtained from 2-chloro-7,8-dihydro-5H-thiopyrano[4,3-b]pyridine-3-carbonitrile (1.75 g, 8.31 mmol) obtained above.
$^1$H NMR ($CDCl_3$, δppm): 3.04 (t, J = 6.2 Hz, 2H), 3.30 (t, J = 6.2 Hz, 2H), 3.81 (s, 2H), 7.68 (d, J = 2.0 Hz, 1H), 8.69 (d, J = 2.0 Hz, 1H).
step 4 In the same manner as in step 1 of Reference Example 17, 7,8-dihydro-5H-thiopyrano[4,3-b]pyridine-3-carboxylic acid hydrochloride (901 mg, 78%) was obtained from 7,8-dihydro-5H-thiopyrano[4,3-b]pyridine-3-carbonitrile (874 mg, 4.96 mmol) obtained above.
$^1$H NMR (DMSO-$d_6$, δppm): 3.01 (t, J = 6.2 Hz, 2H), 3.24 (t, J = 6.2 Hz, 2H), 3.96 (s, 2H), 8.27-8.36(m, 1H), 8.92 (d, J = 1.8 Hz, 1H). ESIMS m/z: $[M-H]^-$ 194.
step 5 In the same manner as in step 3 of Reference Example 2, compound (IX) (79.0 mg, 68%) was obtained as pale-brown crystals from 2-amino-4-(2-furyl)thiazol-5-yl=tetrahydropyran-4-yl=ketone (70.7 mg, 0.254 mmol) described in WO2005/063743 and 7,8-dihydro-5H-thiopyrano[4,3-b]pyridine-3-carboxylic acid hydrochloride (90.9 mg, 0.392 mmol) obtained above.
$^1$H NMR ($CDCl_3$, δppm): 1.81-2.01 (m, 4H), 3.05 (t, J = 6.2 Hz, 2H), 3.15-3.22 (m, 1H), 3.33 (t, J = 6.0 Hz, 2H), 3.51 (ddd, J = 2.9, 11.4, 11.4 Hz, 2H), 3.83 (s, 2H), 4.03-4.10 (m, 2H), 6.53 (dd, J = 1.8, 3.5 Hz, 1H), 7.51 (dd, J = 0.7, 1.8 Hz, 1H), 7.81 (dd, J = 0.7, 3.5 Hz, 1H), 7.94-7.96 (m, 1H), 8.95 (d, J = 2.2 Hz, 1H). ESIMS m/z: $[M+H]^+$ 456.

Reference Example 22

5-Acetyl-N-[4-(2-furyl)-5-(tetrahydropyran-4-carbonyl)thiazol-2-yl]-6-methylpyridine-3-carboxamide (compound (IY))

step 1 In the same manner as in step 2 of Reference Example 2, 5-acetyl-6-methylpyridine-3-carboxylic acid (462 mg, quantitative) was obtained as a yellow solid from ethyl 5-acetyl-6-methylpyridine-3-carboxylate (561 mg, 2.71 mmol) obtained by the method described in Synthesis, vol. 5, p.400 (1986).
$^1$H NMR (DMSO-$d_6$, δppm): 2.63 (s, 3H), 2.66 (s, 3H), 8.54 (d, J - 2.0 Hz, 1H), 9.01 (d, J = 2.0 Hz, 1H).
step 2 2-Amino-4-(2-furyl)thiazol-5-yl=tetrahydropyran-4-yl=ketone (71.2 mg, 0.256 mmol) described in WO2005/063743 was dissolved in DMF (0.5 mL), (benzotriazol-1-yloxy)tripyrrolidinophosphonium hexafluorophosphate (PyBOP) (262 mg, 0.510 mmol), diisopropylethylamine (DIPEA) (150 μL, 0.860 mmol) and 5-acetyl-6-methylpyridine-3-carboxylic acid (93.2 mg, 0.520 mmol) obtained above were added thereto, and the mixture was stirred at 80°C overnight. The mixture was allowed to cool to room temperature, water and a saturated aqueous sodium hydrogen carbonate solution were added thereto and the mixture was extracted with ethyl acetate. The organic layer was washed with saturated brine, and dried over anhydrous magnesium sulfate. The solvent was evaporated under reduced pressure and the obtained residue was purified by silica gel column chromatography (hexane:ethyl acetate=50:50), and reslurried with ethanol-water to give compound (IY) (87.4 mg, 77%) as a pale-yellow solid.
$^1$H NMR ($CDCl_3$, δppm): 1.81-2.01 (m, 4H), 2.67 (s, 3H), 2.86 (s, 3H), 3.13-3.23 (m, 1H), 3.51 (ddd, J = 2.9, 11.4, 11.4 Hz, 2H), 4.03-4.10 (m, 2H), 6.56 (dd, J = 1.7, 3.5 Hz, 1H), 7.55 (dd, J = 0.6, 1.7 Hz, 1H), 7.82 (d, J = 0.6, 3.5 Hz, 1H), 8.54 (d, J = 2.4 Hz, 1H), 9.11 (d, J = 2.4 Hz, 1H). ESIMS m/z: $[M+H]^+$ 440. Reference Example 23

5-Ethyl-N-[4-(3-furyl)-5-(tetrahydropyran-4-carbonyl)thiazol-2-yl]pyridine-3-carboxamide (compound (IZ))

In the same manner as in step 3 of Reference Example 2, compound (IZ) (177 mg, 79%) was obtained as white crystals from 2-amino-4-(3-furyl)thiazol-5-yl=tetrahydropyran-4-yl=ketone (151 mg, 0.541 mmol) obtained by the method described in WO2005/063743 and 5-ethylnicotinic acid (249 mg, 1.64 mmol).
$^1$H NMR ($CDCl_3$, δppm): 1.34 (t, J = 7.6 Hz, 3H), 1.80-2.01 (m, 4H), 2.80 (q, J = 7.6 Hz, 2H), 3.11-3.18 (m, 1H), 3.51

(ddd, J = 2.8, 11.4, 11.4 Hz, 2H), 4.01-4.10 (m, 2H), 7.01 (dd, J = 0.7, 1.8 Hz, 1H), 7.45-7.48 (m, 1H), 8.10-8.13 (m, 1H), 8.63 (dd, J = 0.7, 1.5 Hz, 1H), 8.71-8.76 (m, 1H), 9.02-9.05 (m, 1H). ESIMS m/z: $[M+H]^+$ 412.

Reference Example 24

N-[4-(3-Furyl)-5-(tetrahydropyran-4-carbonyl)thiazol-2-yl]-6,7-dihydro-5H-cyclopenta[b]pyridine-3-carboxamide (compound (IAA))

In the same manner as in step 3 of Reference Example 2, compound (IAA) (71.1 mg, 39%) was obtained as white crystals from 2-amino-4-(3-furyl)thiazol-5-yl=tetrahydropyran-4-yl=ketone (120 mg, 0.432 mmol) and 6,7-dihydro-5H-cyclopenta[b]pyridine-3-carboxylic acid hydrochloride (172 mg, 0.870 mmol).
$^1H$ NMR ($CDCl_3$, δppm): 1.80-2.01 (m, 4H), 2.18-2.30 (m, 2H), 3.03-3.20 (m, 5H), 3.52 (ddd, J = 2.9, 11.3, 11.3 Hz, 2H), 4.01-4.10 (m, 2H), 7.03 (dd, J = 0.6, 2.0 Hz, 1H), 7.48 (dd, J = 1.7, 1.7 Hz, 1H), 8.08-8.10 (m, 1H), 8.68-8.70 (m, 1H), 8.95-8.97 (m, 1H). ESIMS m/z: $[M+H]^+$ 424.

Industrial Applicability

The present invention can be utilized for the treatment and/or prophylaxis of an anxiety disorder such as panic disorder, agoraphobia, obsessive-compulsive disorder, social phobia, posttraumatic stress disorder, particular phobia, generalized anxiety disorder or the like.

## Claims

1. A thiazole derivative represented by the formula (IC)

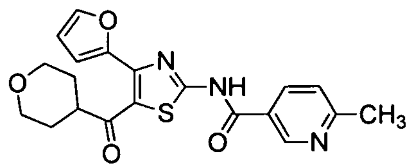

( IC )

or a pharmaceutically acceptable salt thereof for use in the treatment and/or prophylaxis of an anxiety disorder.

2. The thiazole derivative or the pharmaceutically acceptable salt thereof according to claim 1, for use in the treatment and/or prophylaxis of the anxiety disorder selected from panic disorder, agoraphobia, obsessive-compulsive disorder, social phobia, posttraumatic stress disorder, particular phobia and generalized anxiety disorder.

3. Use of the thiazole derivative described in claim 1 or the pharmaceutically acceptable salt thereof, for the production of an agent for the treatment and/or prophylaxis of an anxiety disorder.

4. The use according to claim 3, wherein the anxiety disorder is panic disorder, agoraphobia, obsessive-compulsive disorder, social phobia, posttraumatic stress disorder, particular phobia or generalized anxiety disorder.

## Patentansprüche

1. Thiazol-Derivat dargestellt durch die Formel (IC)

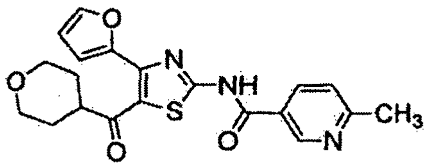

( IC )

oder ein pharmazeutisch verträgliches Salz davon zur Verwendung bei der Behandlung und/oder Vorbeugung einer Angststörung.

2. Thiazol-Derivat oder pharmazeutisch verträgliches Salz davon nach Anspruch 1 zur Verwendung bei der Behandlung und/oder Vorbeugung der Angststörung ausgewählt aus Panikstörung, Agoraphobie, Zwangsstörung, sozialer Phobie, posttraumatischer Belastungsstörung, spezifischer Phobie und generalisierter Angststörung.

3. Verwendung des in Anspruch 1 beschriebenen Thiazol-Derivats oder des pharmazeutisch verträglichen Salzes davon für die Herstellung eines Mittels zur Behandlung und/oder Vorbeugung einer Angststörung.

4. Verwendung nach Anspruch 3, wobei die Angststörung Panikstörung, Agoraphobie, Zwangsstörung, soziale Phobie, posttraumatische Belastungsstörung, spezifische Phobie oder generalisierte Angststörung ist.

**Revendications**

1. Dérivé du thiazole représenté par la formule (IC)

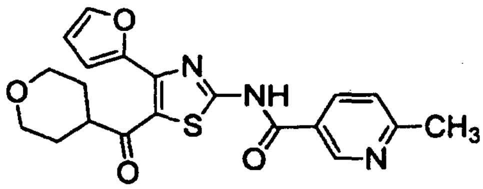

( IC )

ou sel pharmaceutiquement acceptable de celui-ci pour utilisation dans le traitement et/ou la prophylaxie d'un trouble anxieux.

2. Dérivé du thiazole ou sel pharmaceutiquement acceptable de celui-ci selon la revendication 1 pour utilisation dans le traitement et/ou la prophylaxie du trouble anxieux choisi parmi le trouble panique, l'agoraphobie, un trouble obsessionnel compulsif, la phobie sociale, le trouble de stress post-traumatique, une phobie particulière et un trouble anxieux généralisé.

3. Utilisation du dérivé du thiazole selon la revendication 1 ou sel pharmaceutiquement acceptable de celui-ci pour produire un agent pour le traitement et/ou la prophylaxie d'un trouble anxieux.

4. Utilisation selon la revendication 3, dans laquelle le trouble anxieux est le trouble panique, l'agoraphobie, un trouble obsessionnel compulsif, la phobie sociale, le trouble de stress post-traumatique, une phobie particulière ou un trouble anxieux généralisé.

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description


- WO 9401114 A
- WO 2004108137 A
- WO 9912546 A
- EP 1894930 A
- WO 2003022283 A
- WO 2005063743 A
- EP 1700856 A
- WO 2007015528 A
- WO 03043636 A


### Non-patent literature cited in the description


- Diagnostic and Statistical Manual of Mental Disorders. American Psychiatric Association, 1994, 393-444
- *Kaplan & Sadock's Comprehensive textbook of psychiatry,* 1999, 1441
- *Kaplan & Sadock's Comprehensive textbook of psychiatry,* 1999, vol. 1, 1441
- *J. Pharmacol.,* 1995, vol. 68, 119
- *Nature Neuroscience,* 2005, 858
- *Neurology,* 2003, vol. 61 (11), 82-7
- *Nature Reviews Drug Discovery,* 2006, vol. 5, 845
- *Current Pharmaceutical Design,* 2008, vol. 14, 1475
- *Br. J. Pharmacol.,* 2001, vol. 134, 68
- *Ann. Neurol.,* 1998, vol. 43, 507
- *Nature Reviews Drug Discovery,* 2006, vol. 5, 247
- *Progress in Neurobiology,* 2007, vol. 83, 332
- *Naunyn Schmiedebergs Arch Pharmacol.,* 1987, vol. 355 (1), 59
- *Naunyn Schmiedebergs Arch Pharmacol.,* 1987, vol. 355 (2), 204
- *Br. J. Pharmacol.,* 1996, vol. 117 (8), 1645
- **VARANI et al.** *British Journal of Pharmacology,* 1996, vol. 117, 1693
- **BRUNS et al.** *Molecular Pharmacology,* 1986, vol. 29, 331
- *Folia Pharmacologica Japonica,* 2005, vol. 126, 94
- *Eur. J. Pharmacol.,* 2000, vol. 408, 41
- *Folia Pharmacologica Japonica,* 2005, vol. 115, 5
- *Japanese Journal of Clinical Psychopharmacology,* 2006, vol. 9, 2389
- *Folia Pharmacologica Japonica,* 1999, vol. 113, 113
- *Tetrahedron Letters,* 1994, vol. 35, 9355
- *J. Heterocyclic Chem.,* 1987, vol. 24, 351
- *Synthesis,* 1986, vol. 5, 400